# EUROPEAN PATENT SPECIFICATION

(11) **EP 3 993 843 B1**
(45) Date of publication and mention of the grant of the patent: **09.12.2026**
(21) Application number: 20767567.9
(22) Date of filing: 10.09.2020
(51) Int. Cl.: A61L 27/24, A61L 27/38, A61L 27/50, A61L 27/52, C12N 5/00, A61F 2/02, C12N 5/071, A61K 35/39

(54) **MULTI-LAYERED CELL CAPSULES AND USES THEREOF**
MEHRSCHICHTIGE ZELLKAPSELN UND DEREN VERWENDUNGEN
CAPSULES DE CELLULE MULTICOUCHES ET LEURS UTILISATIONS

(30) Priority: 10.09.2019 EP 19382785
(43) Date of publication of application: 11.05.2022
(73) Proprietor: Fundació Institut de Bioenginyeria de Catalunya, 08028 Barcelona (ES)
(72) Inventor: RAMÓN AZCÓN, Javier, 08028 Barcelona (ES); CLUA FERRÉ, Laura, 08028 Barcelona (ES); VELASCO MALLORQUÍ, Ferran, 08028 Barcelona (ES); ORTEGA MACHUCA, Maria Alejandra, 08028 Barcelona (ES); DE CHIARA, Francesco, 08028 Barcelona (ES)
(74) Representative: ZBM Patents - Zea, Barlocci & Markvardsen
(86) International application number: PCT/EP2020/075278
(87) International publication number: WO 2021/048250

(56) References cited:
- WO-A2-2004/047620
- WO-A2-2005/005608
- US-A- 5 529 914
- US-A- 6 020 200
- US-A1- 2014 271 843
- US-B1- 9 283 301
- ROBIN AUGUSTINE: "Skin bioprinting: a novel approach for creating artificial skin from synthetic and natural building blocks", PROGRESS IN BIOMATERIALS, BIOMED CENTRAL LTD, LONDON, UK, vol. 7, no. 2, 12 May 2018 (2018-05-12), pages 77 - 92, XP021259096, ISSN: 2194-0509, DOI: 10.1007/S40204-018-0087-0
- NUREDDIN ASHAMMAKHI ET AL: "Advancing Frontiers in Bone Bioprinting", ADVANCED HEALTHCARE MATERIALS, WILEY - V C H VERLAG GMBH & CO. KGAA, DE, vol. 8, no. 7, 8 February 2019 (2019-02-08), pages n/a, XP072464373, ISSN: 2192-2640, DOI: 10.1002/ADHM.201801048

## Description

This application claims the benefit of European Patent Application EP19382785.4 filed on September 10th, 2019.

### Technical Field

The present invention relates to capsules for cell encapsulation. In particular, the invention relates to multi-layered hydrogel cell capsules that increase cell viability and biocompatibility. The capsules of the invention are particularly useful for encapsulating pancreatic islets.

### Background Art

Hydrogel capsules are under strong investigation for the encapsulation of living cells for tissue engineering and regenerative medicine due to their relatively low cytotoxicity and similar structure to extracellular matrix. They are designed to allow the diffusion of oxygen and nutrients and the release of the therapeutic proteins secreted by the encapsulated cells. Importantly, they must also be able to ward off recognition by the host immune system.

Non-specific host response is one major challenge to clinical application of encapsulated cells. This reaction involves the recruitment of early innate immune cells such as neutrophils and macrophages, followed by fibroblasts which deposit collagen to form a fibrous capsule surrounding the implanted object. The fibrotic cellular overgrowth on the capsules cuts off the diffusion of oxygen and nutrients and lead to necrosis of encapsulated cells, thus leading to the eventual failure of many implantable medical devices such as encapsulated pancreatic islets.

Most commonly, hydrogel capsules for cell encapsulation are based on a monolayer of alginate hydrogel. One challenge of this type of capsules is their biocompatibility. In fact, alginate has revealed low biocompatible properties, which does not induce effective cell attachment or proliferation.

When used to encapsulate islets, alginate capsules also present the problem of incomplete coverage of the islets. Islets protruding outside the capsules are more frequently observed when their number density in alginate solution increases or the capsule size decreases, both of which are desirable to minimize the transplantation volume. It has been recognized that incomplete coverage would not only cause the rejection of exposed cells but may also allow the infiltration of macrophages and fibroblasts into the capsules through the exposed areas.

A double encapsulation process has been proposed wherein a two-fluid co-axial electrojetting system allows the formation of two-layer alginate capsules. However, the materials and method of synthesis used do not provide a clear core-shell structure wherein the two layers of the capsule do not mix, thereby reducing cell viability and biocompatibility. Moreover, the double encapsulation methods often involve multiple steps which cause damage to islets and it is not clear whether the coatings are sufficiently robust for clinical use.

US 6 020 200 describes cells which are encapsulated in a composition of alginate which is then cured by treatment with a divalent cation solution. These beads are coated with a collagen matrix which is crosslinked to further stabilize their structure.

In summary, despite promising studies in various animal models over many years, encapsulated human cells so far have not made an impact in the clinical setting. Many non-immunological and immunological factors such as biocompatibility, reduced immunoprotection, hypoxia, pericapsular fibrotic overgrowth, effects of the encapsulation process, and post-transplant inflammation hamper the successful application of this promising technology.

Therefore, there is still a need for capsules for encapsulating cells that mimic the complexity of the cellular native environment while efficiently prevent the immune system attacks.

### Summary of Invention

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

The present inventors have developed a novel type of hydrogel capsules for cell encapsulation that improve cellular viability and biocompatibility.

As shown in the examples below, the inventor has surprisingly found that hydrogel cell capsules comprising collagen that has been covalently crosslinked with tannic acid only on its external layer provide enhanced cell viability, reduced capsule degradation, and efficient immune evasion.

Unexpectedly, the inventors found that a double encapsulation process wherein a single material is used to generate two-layer capsules through different cross-linking methods allows the formation of a clear core-shell structure where there is no risk of cells protruding to the outside.

The remarkable advantages shown by the novel capsules herein provided are clear: they provide a core nucleus extremely similar structure to the extracellular matrix while providing an outer surface that allows an efficient protection of cells while allowing metabolites exchange. Importantly, as shows in the examples below the inventors also found that the formation of an outer protective shell surprisingly enhances the insulin production of encapsulated islet cells.

Furthermore, the inventors have found that these novel capsules provide an optimal environment for cell differentiation, particular for cell types that form aggregates. Thus, when transdifferentiating or differentiation methods are carried out inside the capsules of the invention, the speed an efficient of the process is highly improved.

The use of a single natural material as the main component of the two layers of the capsule greatly facilitates their synthesis, thereby avoiding multiple and complex steps that can affect cell viability. The material of the capsules herein provided in combination with their porous size cut notably the time between glucose sensing and the release of insulin. At same time, they provide efficient protection from host immune cells.

Also, when used to embed pancreatic islets, the biodegradable protein forming the core layer of the capsules, i.e. collagen, easily adapts itself to cellular clustering and growth. In addition, the cells are confined within a non- biodegradable crosslinked coating that prevents pancreas islets dispersion, but at same time does not affect the formation of new capillaries. This new complex system is the key to achieving better pancreatic islets performances, thanks to the integration of nanotechnology, biology and tissue engineering.

In view of the above, the new cells capsules herein provided constitutes a great advance in the field of medicine, in particular for the treatment of disorders that require cell implants.

Thus, in a first aspect, the invention provides a hydrogel capsule comprising a cell, a protein, and a cross-linking agent, wherein the cell is within a first core layer comprising the protein, and wherein the first core layer is surrounded by a second layer comprising the protein and the cross-linking agent, and wherein the cross-linking agent is tannic acid and the protein comprises collagen.

The inventors have also developed novel implants formed by embedding the capsules above indicated in a microporous scaffold, which greatly facilitates their handling, implantation, and retrieval.

Thus, in a second aspect, the invention provides an implant comprising the hydrogel capsule according to the first aspect and a microporous scaffold.

In a third aspect, the invention provides the hydrogel capsule according to the first aspect or the implant according to the second aspect for use in therapy, diagnosis or prognosis.

In a fourth aspect, the invention provides the use of the hydrogel capsule as defined in the first aspect or the implant as defined in the second aspect for the in vitro culture of cells.

In a fifth aspect, the invention provides an ex vivo method for differentiating an undifferentiated cell to an islet cell, or alternatively, for transdifferentiating a differentiated cell to an islet cell, comprising the steps of (a) producing a hydrogel capsule as defined in the first aspect wherein the cell is the undifferentiated or differentiated cell; (b) contacting the hydrogel capsule produced in (a) with a factor selected from the group consisting of KGF, SANT1, retinoic acid, and mixtures thereof.

In a sixth aspect, the invention provides a method for producing a hydrogel capsule as defined in the first aspect, the method comprising the steps of (a) forming the first core layer comprising the protein and the cell; (b) allowing non-covalent reticulation of the protein to form a hydrogel; and (c) submerging the hydrogel in a solution comprising the crosslinking agent.

### Brief Description of Drawings

Figure 1 shows NMR - ¹H spectrum of collagen and collagen methacrylate. a) represents methyl signal of methacrylate; b) represents signals of oleofinic protons; and c) represents signal of lysine. H) represents high; M) represents medium, L) represents low; and C) represents control.
Figure 2 shows the degradation rate of the hydrogel capsules of the invention in presence of 0.25 U/ml of collagenase type I. Capsules of collagen methacrylate (ColMA, circles), collagen treated with tannic acid (ColTA, squares) and collagen reticulated with temperature (Collagen, triangles) were tested. Y-axis represents the Degradation (%), and the x-axis represents the time (h).
Figure 3 shows SEM images of collagen methacrylate (ColMA), collagen treated with tannic acid (ColTA) and collagen reticulated with temperature (Collagen). Scale bar = 3 µm.
Figure 4 shows rheometry analysis of the three materials, collagen methacrylate (ColMA, circles), collagen treated with tannic acid (ColTA, squares) and collagen reticulated with temperature (Collagen, triangles). Y-axis represents the Stiffness (Pa), and the x-axis represents the Frequency (Hz).
Figure 5 shows a picture of the morphology of a capsule formed only by collagen (left) and the capsule of the invention formed by collagen crosslinked with tannic acid (right). The capsule of the invention maintains a perfectly round morphology and can be easily handled.
Figure 6 shows the stiffness of the hydrogels formed by collagen crosslinked with tannic acid solution. The x-axis represents the position (in mm), and the y-axis represents the stiffness (KPa).
Figure 7 shows the quantification of porous diameter by ImageJ software obtained from SEM images, pristine collagen (control), collagen crosslinked with tannic acid 1% w/t (T.A 1x) and collagen crosslinked with tannic acid 3% w/t (T.A 3x). The y-axis represents the Feret diameter (µm).
Figure 8 shows a live staining to assess cell viability by labeling cells with CFDA-SE in hydrogel capsules fabricated in collagen and in collagen treated with tannic acid (ColTA) (live cells in green in the original picture). Images recorded after 15 days of encapsulation.
Scale bar = 500 µm. Arrow marks the increased number of cells in ColTA capsules.
Figure 9 shows a cell proliferations assessment with Alamar blue and MTS. Cell density was 7 x 10⁶ cells/mL at day 0 and cultured up to 30 days. Grey bars represent collagen capsules and white bars represent the ColTA capsules of the invention. The left y-axis represents Alamar blue (570 nm) and the right y-axis represents MTS (510 nm).
Figure 10 shows a cell escaping assay. At day 0, each spheroid (Cell density was 7 x 10⁶ cells/mL) was placed in a 96 well-plate. At indicated time points, the spheroids were removed and placed in a new well-plate while the well was treated with trypsin-EDTA to detach the escaped cells from the bottom of the well. The cell counting was performed using an automated cell counter Countess^{™} (15397802, fisher scientific). The y-axis represents number of cells.
Figure 11 A and B shows the live staining and immunostaining of insulin in hydrogel capsules fabricated in collagen and in collagen treated with tannic acid (CoITA). Images recorded after 15 days of encapsulation. Scale bar of 500 µm. Arrow marks the increased amount of insulin produced by cells in ColTA capsules.
Figure 12 shows the insulin quantification of GSIS assay of collagen spheroids (control) and collagen crosslinked spheroids with tannic acid 1x (ColTA 1x). The y-axis represents Insulin secretion (ng of insulin).

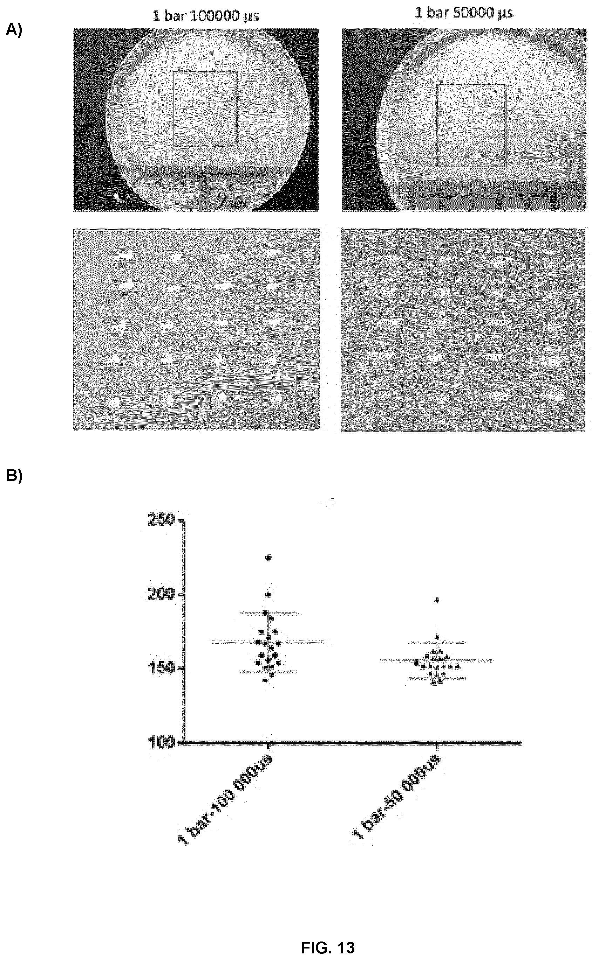

Figure 13 shows on A) macroscopic picture of the hydrogels using 2 different bioprinting settings. B) the spheroids bio-printed using the open valve time of 50000 µs, showed a smaller with 100000 µs with a lower intra-group variability. The y-axis represents diameter (µm).
Figure 14 shows, on the left panel, representative images of 3D spheroid cell distribution in collagen and collagen plus tannic acid. On the right panel, confocal images of live (green in the original) and dead (red in the original) cells within the 2 different matrices. Scale bar is 200 µm.
Figure 15 shows on the left panel, representative confocal images of 3D spheroids containing hepatocytes labelled for albumin (green in the original). On the right panel, DAPI (blue in the original) counterstaining the nuclei. Scale bar is 200 µm.
Figure 16 shows the pore distribution of microporous scaffolds with different concentration of carboxymethylated cellulose. The y-axis represents the pore diameter in µm, and the x-axis represents the concentration of carboxymethylated cellulose.
Figure 17 shows the swelling ratio of microporous scaffolds produced with the indicated compounds. The y-axis represents swelling ratio (%).
Figure 18 shows the stiffness measurements obtained by compression assays of different compounds used for producing the microporous scaffolds. The y-axis represents stiffness (KPa).
Figure 19 shows a SEM image of a carboxymethylated cellulose cryogel. Scale bar = 300 µm.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the," also include the plural of the noun.

"Capsule," as used herein, refers to a particle formed of a hydrogel, having a non-covalent crosslinked core (or nucleus) that is surrounded by a layer that is covalently cross-linked, thereby forming a protective shell. The capsule may have any shape suitable for cell encapsulation. The capsules of the invention contain one or more cells in the core layer, thereby "encapsulating" the cells. The term "core" refers to the discrete inner part of the capsule that is not in contact with the exterior.

As used herein, "hydrogel" refers to a substance formed when a protein or protein fragment is cross-linked via covalent, ionic, or hydrogen bonds to create a three-dimensional open-lattice structure which entraps water molecules to form a gel. Biocompatible hydrogel refers to a polymer that forms a gel which is not toxic to living cells and allows sufficient diffusion of oxygen and nutrients to the entrapped cells to maintain viability. In the present invention the hydrogel is formed by collagen.

A "protein" as used herein, refers to a polymer made up of amino acids. According to the claimed invention, the

The term "cross-linking agent" refers to a monomer containing at least two reactive groups capable of forming covalent linkages with the protein that forms the hydrogel. The cross-linking agent used to form the hydrogel capsules according to the claimed invention is tannic acid.

As used herein, the term "collagen" refers to a family of homotrimeric and heterotrimeric proteins comprised of collagen monomers. There are a multitude of known collagens which serve a variety of functions in the body. There are an even greater number of collagen monomers, each encoded by a separate gene, that are necessary to make the different collagens. The most common collagens are types I, II, and III. Collagen molecules contain large areas of helical structure, wherein the three collagen monomers form a triple helix. The regions of the collagen monomers in the helical areas of the collagen molecule generally have the sequence G-X-Y, where G is glycine and X and Y are any amino acid, although most commonly X and Y are proline and/or hydroxyproline. Any collagen can be used to generate the hydrogel capsules of the invention.

As used herein, the term "fibrillar collagen" means a collagen of a type which can normally form collagen fibrils. The fibrillar collagens are collagen types I-III, V, and XI. The term fibrillar collagen encompasses both native (i.e., naturally occurring) and variant fibrillar collagens (ie., fibrillar collagens with one or more alterations in the sequence of one or more of the fibrillar collagen monomers).

"Cell," as used herein, refers to individual cells, cell aggregates, or organoids. Cells can be, for example, xenogeneic, autologous, or allogeneic. Cells can also be primary cells. Cells can also be cells derived from the culture and expansion of a cell obtained from a subject. For example, cells can also be stem cells or derived from stem cells. Cells can also be immortalized cells. Cells can also be genetically engineered to express or produce a protein, nucleic acid, or other product. Cells can be differentiated from reprogrammed cells or transdifferentiated from differentiated cells.

As used herein, "cell transdifferentiation" refers to a process where one mature differentiated cell switches its phenotype and function to that of another mature differentiated cell type without undergoing an intermediate pluripotent state or becoming a progenitor cell. The term "cell reprogramming" refers to the conversion of a differentiated cell with restricted developmental potential to a pluripotent cell. The basic difference between reprogramming and transdifferentiation is the following: (i) Reprogramming requires a reversal change of a differentiated cell into a pluripotent stem cell (i.e. iPS), which next may undergo a differentiation process into another differentiated cell. (ii) Transdifferentiation does not require a full reversal into iPS cells in order to transform into another cell type. It is the direct conversion of one adult cell into another cell type without undergoing into a pluripotent stem cell state. Whereas iPS cell reprogramming is a rather time-consuming process, transdifferentiation is often fast and highly efficient.

"Autologous", as used herein, refers to a transplanted cell taken from the same individual. "Allogeneic" refers to a transplanted cell taken from a different individual of the same species. "Xenogeneic" refers to a transplanted cell taken from a different species.

As used herein, the term "organoid" refers to structures resembling whole organs that have been generated from stem cells or undifferentiated, through three-dimensional culture systems, such as the three-dimensional hydrogel of the invention. Organoids can be also derived from isolated organ progenitors.

The term "microporous scaffold" refers to a biocompatible polymeric material that contains an array of pores of similar or different sizes that are substantially connected.

As used herein, the term "cryogel" refers to microporous scaffolds formed by a process that includes freeze-drying a gel solution.

"Anti-inflammatory drug" refers to a drug that directly or indirectly reduces inflammation in a tissue. The term includes, but is not limited to, drugs that are immunosuppressive. The term includes anti-proliferative immunosuppressive drugs, such as drugs that inhibit the proliferation of lymphocytes. "Immunosuppressive drug" refers to a drug that inhibits or prevents an immune response to a foreign material in a subject. Immunosuppressive drugs generally act by inhibiting T-cell activation, disrupting proliferation, or suppressing inflammation. A person who is undergoing immunosuppression is said to be immunocompromised.

As used herein, the term "size" refers to a characteristic physical dimension. For example, in the case of a capsule that is substantially spherical, the size of the capsule corresponds to the diameter of the capsule. When referring to a set of capsule as being of a particular size, it is contemplated that the set can have a distribution of sizes around the specified size. Thus, as used herein, a size of a set of capsule can refer to a mode of a distribution of sizes, such as a peak size of the distribution of sizes. In addition, when not perfectly spherical, the diameter is the equivalent diameter of the spherical body including the object. This diameter is generally referred as the "hydrodynamic diameter", which measurements can be performed using a Wyatt Möbius coupled with an Atlas cell pressurization system or Malvern. Transmission Electron Microscopy (TEM) or Scanning Electron Microscopy (SEM) images do also give information regarding diameters.

As used herein, the term "% w/w", "wt%", or "percentage by weight" of a component refers to the amount of the single component relative to the total weight of the composition or, if specifically mentioned, of other component.

As used herein, "companion diagnostic methods" are assays used to identify subjects susceptible to treatment with a particular drug, to monitor treatment, and/or to identify an effective dosage for a subject or sub-group of subjects. Companion diagnostics may be useful for stratifying patient disease, disorder or condition severity levels, allowing for modulation of treatment regimen and dose to reduce costs, shorten the duration of clinical trial, increase safety and/or increase effectiveness. Companion diagnostics may be used to predict the development of a disease, disorder or condition and aid in the prescription of preventative therapies. Some companion diagnostics may be used to select subjects for one or more clinical trials. In some cases, companion diagnostic assays may go hand-in-hand with a specific treatment to facilitate treatment optimization.

As mentioned above, in a first aspect the present invention provides a two-layer capsule comprising a cell, a protein polymer, and a cross-linking agent, wherein the cell is within a first core layer comprising the protein; and wherein the first core layer is surrounded by a second layer comprising the protein and the cross-linking agent, wherein the cross-linking agent is tannic acid and the protein comprises collagen.

The capsules of the invention are formed by an inner core or nucleus, which contains the cells embedded in the hydrogel structure formed by the non-covalent bonding of the protein units. Surrounding the inner core, there is an outer shell formed by the protein, which is further cross-linked in a covalent way with a cross-linking agent. Therefore, the capsules herein provided present a core layer that is a cell-friendly layer that promotes cell viability, and a second layer that protects the capsules from degradation and the attacks of the immune system. The two layers are formed by the same hydrogel-forming protein.

According to the claimed invention, the protein comprises collagen. In a more particular embodiment, the collagen is fibrillar collagen. In a more particular embodiment, the fibrillar collagen is collagen type I.

In another embodiment, optionally in combination with any of the embodiments provided above or below, the collagen is selected from the group consisting of pure collagen, collagen derivative, collagen hydrolysate, mixtures comprising collagen and extracellular matrix proteins, and combinations thereof. One mixture of proteins containing collagen that is suitable for producing the capsules of the invention is Matrigel^{™} (BD Biosciences).

According to the claimed invention, the cross-linking agent is tannic acid. The cross-linking of the proteins is carried out with techniques known to those skilled in the art. For instance, the skilled in the art would know that tannic acid directly reacts with the protein residues.

The cross-linking agent is externally applied to the capsule after the formation of the hydrogel through the non-covalent reticulation of the protein, said non-covalent reticulation for example by heat treatment. In this way, the resulting capsule is formed by a non-covalent cross-linked protein with an external layer that is, in addition, covalently crosslinked. This double reticulated structure provides the capsules of the invention with optimal properties for cell function and biocompatibility.

In a particular embodiment, the second layer is collagenase resistant. In a more particular embodiment, the second layer resists the degradation with collagenase for more than 2 days.

In a particular embodiment, the stiffness of the capsule is from 800 Pa to 16000 Pa, as measured by parallel plate rheometry. More particularly, from 882 Pa to 15184 Pa. In another particular embodiment, the stiffness of the core layer is from 500 Pa to 1000 Pa, from 700 Pa to 900 Pa, or 882 Pa, as measured by parallel plate rheometry. In another particular embodiment, the stiffness of the second layer is from 10000 Pa to 20000 Pa, from 14000 Pa to 16000 Pa, or 15184 Pa, as measured by parallel plate rheometry. These viscoelasticity values may favor cell survival.

Mechanical properties of hydrogels were assessed using parallel plate rheometry (Discovery HR-2 rheometer, TA instruments, Inc., UK). Hydrogels were fabricated in cylindrical shape (1mm thick, 8 mm diameter) and bulk modulus (G') and viscous modulus (G") measurements were recorded at a frequency range of 1-10 Hz at room temperature using 8mm aluminum plate geometry. The gap was adjusted starting from the original sample height and compressing the sample to reach a normal force of 0.3N. Rheological measurements were made on hydrogels after 24h post gelation.

In a particular embodiment optionally in combination with any of the embodiments provided above or below, the porous size of the second layer of the capsules is smaller than 5 µm. In a particular embodiment, it is smaller than 200 nm. In a more particular embodiment, it is from 50 nm to 200 nm. In an even more particular embodiment, it is from 80 nm to 120 nm. These porous sizes allow the interchange of oxygen and nutrients while not allowing the penetrance of immune cells.

The porous size is determined by the concentration of cross-linking agent in the second layer. Thus, in a particular embodiment optionally in combination with any of the embodiments provided above or below, the cross-linking agent is present in the second layer at a concentration from 0.1 to 4 % w/w, more particularly from 0.3 to 3.5 % w/w, or more particularly from 0.5 to 3% w/w.

The second layer should present an appropriate thickness in order to efficiently protect the capsule from degradation. In a particular embodiment of the first aspect, optionally in combination with any of the embodiments provided above or below, the second layer has a thickness from 5 µm to 50 µm, more particularly from 10 µm to 25 µm.

According to the first aspect of the present invention, the capsule comprises a cell, collagen, and tannic acid, wherein the cell is within a first core layer comprising the collagen; and wherein the first core layer is surrounded by a second layer comprising the collagen and the tannic acid.

In a particular embodiment of the first aspect, optionally in combination with any of the embodiments provided above or below, the capsules have a mean diameter from 200 µm to 3 mm. More particularly from 400 µm to 2 mm. More particularly from 450 µm to 1 mm. Even more particularly, from 500 µm to 750 µm. The size is controlled by the volume of the hydrogel deposited in the super hydrophobic substrate. This volume is controlled by the aperture of the piezoelectric valve in the printer. All these data have been calculated and we have a relation within the aperture time of the valve and size (see 3D printing methodology below).

In a particular embodiment of the first aspect, optionally in combination with any of the embodiments provided above or below, the porous size of the second layer of the capsules is smaller than 5 µm, the capsules have a mean diameter from 200 µm to 3 mm, and the stiffness of the capsule is from 800 Pa to 16000 Pa, as measured by parallel plate rheometry.

In a particular embodiment of the first aspect, optionally in combination with any of the embodiments provided above or below, the capsules have a shape, wherein the shape is selected from a group consisting of a sphere, sphere-like shape, spheroid, spheroid-like shape, ellipsoid, ellipsoid-like shape, stadiumoid, stadiumoid-like shape, disk, disk-like shape, cylinder, cylinder-like shape, rod, rod-like shape, cube, cube-like shape, cuboid, cuboidlike shape, torus, torus-like shape, flat surface, curved surfaces, or combinations thereof. In a more particular embodiment, the capsules have a shape selected from sphere or spheroid.

The cell type chosen for encapsulation in the disclosed compositions depends on the desired therapeutic effect. The cell may be from the patient (autologous cells), from another donor of the same species (allogeneic cells), or from another species (xenogeneic). Xenogeneic cells are easily accessible, but the potential for rejection and the danger of possible transmission of viruses to the patient restricts their clinical application. Anti-inflammatory drugs combat the immune response elicited by the presence of such cells. In the case of autologous cells, the anti-inflammatory drugs reduce the immune response provoked by the presence of the foreign hydrogel materials or due to the trauma of the transplant surgery. Cells can be obtained from biopsy or excision of the patient or a donor, cell culture, or cadavers. Evidently, mixtures of different cell types can also be encapsulated.

In some embodiments, the cell secretes a therapeutically effective substance, such as a protein or nucleic acid. In some embodiments, the cell metabolizes toxic substances. In some embodiments, the cell forms structural tissues, such as skin, bone, cartilage, blood vessels, or muscle. In some embodiments, the cell is natural, such as islet cells that naturally secrete insulin, or hepatocytes that naturally detoxify. In some embodiments, the cell is genetically engineered to express a heterologous protein or nucleic acid and/or overexpress an endogenous protein or nucleic acid.

Thus, in a particular embodiment, optionally in combination with any of the embodiments provided above or below, the cell is selected from the group consisting of pancreatic cell, hepatic cell, cardiovascular cell, nerve cell, muscle cell, cartilage cell, bone cell, skin cell, hematopoietic cell, immune cell, stem cell, genetically engineered cell reprogrammed cell, and mixtures therefor.

In a more particular embodiment, optionally in combination with any of the embodiments provided above or below, the cells are hormone-producing cells. Hormone-producing cells can produce one or more hormones, such as insulin, parathyroid hormone, anti-diuretic hormone, oxytocin, growth hormone, prolactin, thyroid stimulating hormone, adrenocorticotropic hormone, follicle-stimulating hormone, lutenizing hormone, thyroxine, calcitonin, aldosterone, Cortisol, epinephrine, glucagon, estrogen, progesterone, and testosterone.

In a more particular embodiment, optionally in combination with any of the embodiments provided above or below, the cell is a pancreatic cell. In an even more particular embodiment, the pancreatic cell is an islet cell. In an even more particular embodiment, the islet cell is a beta cell.

In a more particular embodiment, optionally in combination with any of the embodiments provided above or below, the cell is a hepatic cell. When the capsules of the invention comprise hepatic cells, they can be used for treating patients with hepatic problems, for example, a subject with a hepatic dysfunction can be implanted with the capsules or implants of the invention which will act as an artificial liver thereby performing hepatic dialysis.

Types of cells for encapsulation in the disclosed hydrogel capsules include cells from natural sources, such as cells from xenotissue, cells from a cadaver, and primary cells; stem cells, such as embryonic stem cells, mesenchymal stem cells, and induced pluripotent stem cells; derived cells, such as cells derived from stem cells, cells from a cell line, reprogrammed cells, reprogrammed stem cells, cells derived from reprogrammed stem cells, and transdifferentiated cells; and genetically engineered cells, such as cells genetically engineered to express a protein or nucleic acid, cells genetically engineered to produce a metabolic product, and cells genetically engineered to metabolize toxic substances. Thus, in a more particular embodiment, optionally in combination with any of the embodiments provided above or below, the cell is a reprogrammed cell or a transdifferentiated cell.

Cells can be obtained directly from a donor, from established cell culture lines, or from cell culture of cells from a donor. In some particular embodiments, cells are obtained directly from a donor, washed and implanted directly in combination with the protein material. In other particular embodiments, cells are obtained from the donor, reprogrammed *in vitro* to pluripotent stem cell and then differentiated into the desired cell type and then encapsulated. In other particular embodiments, cells are obtained from the donor, reprogrammed *in vitro* to pluripotent stem cell, the stem cells are then encapsulated and later differentiated into the desired cell type within the capsule. In other particular embodiments, differentiated cells are obtained from the donor, transdifferentiated *in vitro* into the desired cell type, and then encapsulated. In other particular embodiments, differentiated cells are obtained from the donor and directly encapsulated, and then differentiated into the desired cell type within the capsule. The cells are cultured, reprogrammed, differentiated, or transdifferentiated using techniques known to those skilled in the art of cell and tissue culture. In particular, various methods of cell transdifferentiation or reprogrammed are known in the art (Zhou, Q., et al., "In vivo reprogramming of adult pancreatic exocrine cells to b -cells", 2008, Nature, vol. 455(7213), pp. 627-32).The transdifferentiated cells may optionally be cultured prior to encapsulation or using any suitable method of culturing islet cells as is known in the art.

Thus, in a more particular embodiment of the first aspect, optionally in combination with any of the embodiments provided above or below, the cell is a differentiated cell, a pluripotent stem cell or a transdifferentiated cell.

It was surprisingly found by the present inventors that the capsules of the invention improved the efficiency of the differentiation or transdifferentiation processes of cells into islet cells.

Cell viability can be assessed using standard techniques, such as histology and fluorescent microscopy. The function of the encapsulated cells can be determined using a combination of these techniques and functional assays. For example, pancreatic islet cells and other insulin-producing cells can be implanted to achieve glucose regulation by appropriate secretion of insulin. Other endocrine tissues and cells can also be implanted.

The amount and density of cells encapsulated in the disclosed hydrogel capsules vary depending on the choice of cell, hydrogel, and site of implantation.

Thus, in a particular embodiment, optionally in combination with any of the embodiments provided above or below, the capsule comprises cells at a concentration from 0.1 x 10⁶ to 10 x 10⁶ cells/ml, more particularly from 0.5 x 10⁶ to 2 x 10⁶ cells/ml, and even more particularly 1 x 10⁶ cells/ml

In other particular embodiments, the cells are forming cell aggregates or organoids. For example, islet cell aggregates (or whole islets) contain from 50 to 1000 cells for each aggregate of 150 µm diameter, which is defined as one islet equivalent (IE). Therefore, in some embodiments, islet cells are present at a concentration from 50 to 10000 IE/ml, particularly from 200 to 3000 IE/ml, more particularly from 500 to 750 IE/ml.

In some embodiments, the disclosed capsules contain cells genetically engineered to produce a therapeutic protein or nucleic acid. In these embodiments, the cell can be a stem cell (e.g., pluripotent), a progenitor cell (e.g., multipotent or oligopotent), or a terminally differentiated cell (i.e., unipotent). The cell can be engineered to contain a nucleic acid encoding a therapeutic polynucleotide such miRNA or RNAi or a polynucleotide encoding a protein. The nucleic acid can be integrated into the cells genomic DNA for stable expression or can be in an expression vector (e.g., plasmid DNA). The therapeutic polynucleotide or protein can be selected based on the disease to be treated and the site of transplantation. In some embodiments, the therapeutic polynucleotide or protein is anti-neoplastic. In other embodiments, the therapeutic polynucleotide or protein is a hormone, growth factor, or enzyme.

Therapeutic agents for secretion by genetically engineered cells include, for example, insulin, glucagon, thyroid stimulating hormone; beneficial lipoproteins such as Apol; prostacyclin and other vasoactive substances, anti-oxidants and free radical scavengers; soluble cytokine receptors, for example soluble transforming growth factor (TGF) receptor, or cytokine receptor antagonists, for example ILIra; soluble adhesion molecules, for example ICAM-1 ; soluble receptors for viruses, e.g. CD4, CXCR4, CCR5 for HIV; cytokines; elastase inhibitors; bone morphogenetic proteins (BMP) and BMP receptors 1 and 2; endoglin; serotonin receptors; tissue inhibiting metalloproteinases; potassium channels or potassium channel modulators; anti-inflammatory factors; angiogenic factors including vascular endothelial growth factor (VEGF), transforming growth factor (TGF), hepatic growth factor, and hypoxia inducible factor (HIF); polypeptides with neurotrophic and/or anti-angiogenic activity including ciliary neurotrophic factor (CNTF), glial-derived neurotrophic factor (GDNF), nerve growth factor (NGF), brain-derived neurotrophic factor (BDNF), neurotrophin-3, nurturin, fibroblast growth factors (FGFs), endostatin, ATF, fragments of thrombospondin, variants thereof and the like. More preferred polypeptides are FGFs, such as acidic FGF (aFGF), basic FGF (bFGF), FGF-1 and FGF-2 and endostatin.

In some particular embodiments, the secreted agent is a protein or peptide. Examples of protein active agents include, but are not limited to, cytokines and their receptors, as well as chimeric proteins including cytokines or their receptors, some of them previously mentioned and including, for example tumor necrosis factor alpha and beta, their receptors and their derivatives; renin; lipoproteins; colchicine; prolactin; corticotrophin; vasopressin; somatostatin; lypressin; pancreozymin; leuprolide; alpha- 1 -antitrypsin; clotting factors such as factor VIIIC, factor IX, tissue factor, and von Willebrand factor; anti-clotting factors such as Protein C; atrial natriuretic factor; lung surfactant; a plasminogen activator other than a tissue-type plasminogen activator (t-PA), for example a urokinase; bombesin; thrombin; hemopoietic growth factor; enkephalinase; RANTES (regulated on activation normally T-cell expressed and secreted); human macrophage inflammatory protein (MIP-1 -alpha); a serum albumin such as human serum albumin; mullerian-inhibiting substance; relaxin A-chain; relaxin B-chain; prorelaxin; mouse gonadotropin-associated peptide; chorionic gonadotropin; a microbial protein, such as beta-lactamase; DNase; inhibin; activin; receptors for hormones or growth factors; integrin; protein A or D; rheumatoid factors; platelet-derived growth factor (PDGF); epidermal growth factor (EGF); transforming growth factor (TGF) such as TGF-a and TGF-β, including TGF-βI, TGF- 2, TGF- 3, TGF- 4, or TGF- 5; insulin-like growth factor-I and -II (IGF-I and IGF-II); des(I-3)- IGF-I (brain IGF-I), insulin-like growth factor binding proteins; CD proteins such as CD- 3, CD-4, CD-8, and CD- 19; erythropoietin; osteoinductive factors; immunotoxins; an interferon such as interferon-alpha (e.g., interferon. alpha.2 A), -beta, -gamma, -lambda and consensus interferon; colony stimulating factors (CSFs), e.g., M-CSF, GM-CSF, and G-CSF; interleukins (ILs), e.g., IL-1 to IL-10; superoxide dismutase; T-cell receptors; surface membrane proteins; decay accelerating factor; transport proteins; homing receptors; addressins; fertility inhibitors such as the prostaglandins; fertility promoters; regulatory proteins; antibodies (including fragments thereof) and chimeric proteins, such as immunoadhesins; precursors, derivatives, prodrugs and analogues of these compounds, and pharmaceutically acceptable salts of these compounds, or their precursors, derivatives, prodrugs and analogues. Suitable proteins or peptides may be native or recombinant and include, e.g., fusion proteins. Hormones to be included in the disclosed hydrogel capsules or, most preferably, produced from cells included in the disclosed hydrogel capsules can be any hormone of interest. The disclosed capsules can also be used to provide vaccine components. For example, cells expressing vaccine antigens can be included in the hydrogel capsule. The disclosed hydrogel capsules can also be used to provide antibodies. For example, cells expressing antibodies can be included in the hydrogel capsule.

The site, or sites, where cells are to be implanted is determined based on individual need, as is the requisite number of cells. For cells replacing or supplementing organ or gland function (for example, hepatocytes or islet cells), the mixture can be injected into the mesentery, subcutaneous tissue, retroperitoneum, preperitoneal space, and intramuscular space.

The invention also provides a hydrogel capsule comprising a cell; a protein; and a cross-linking agent; wherein the cell is within a first core layer comprising the protein; wherein the first core layer is surrounded by a second layer comprising the protein and the cross-linking agent; wherein the protein comprises collagen and the cross-linking agent is tannic acid, wherein the porous size of the second layer of the capsules is smaller than 5 µm, and wherein the capsule has a mean diameter from 200 µm to 3 mm.

As mentioned above, in a second aspect the invention provides an implant comprising the capsule of the invention and a microporous scaffold.

The inventors have found that embedding the capsules of the invention in a microporous scaffold facilitates their handling and implantation into the patient.

In a particular embodiment of the second aspect, optionally in combination with any of the embodiments provided above or below, the microporous scaffold comprises a compound selected from the group consisting of polysaccharides (e.g. cellulose, carboxymethyl cellulose, nano-fibrilated cellulose, agarose, or alginate), collagen, gelatin, polyphosphazenes, polyethylene glycol, poly(acrylic acids), poly(methacrylic acids), copolymers of acrylic acid and methacrylic acid, poly(alkylene oxides), poly(vinyl acetate), polyvinylpyrrolidone (PVP), and copolymers and blends thereof. More particularly, the polysaccharide is selected from cellulose, carboxymethyl cellulose, nano fibrillated cellulose, agarose, alginate, and mixtures thereof.

In a particular embodiment of the second aspect, optionally in combination with any of the embodiments provided above or below, the microporous scaffold comprises carboxymethyl cellulose from 0.25 to 5 % w/w or from 0.5 to 1 % w/w. In a more particular embodiment, the microporous scaffold is a cryogel. In an even more particular embodiment, the microporous scaffold is a cryogel of carboxymethyl cellulose at 0.5 % w/w.

In a more particular embodiment, the porous of the microporous scaffold have a mean diameter from 10 µm to 350 µm. More particularly, from 20 µm to 150 µm. In an even more particular embodiment, the microporous scaffold is formed by two horizontal layers with different porous size. In a more particular embodiment, the porous size of the lower layer is from 20 µm to 100 µm, and the porous size of the upper layer is from 20 µm to 150 µm. This double layer structure allows the efficient retention of the capsules inside the scaffold.

In a particular embodiment, the stiffness of the microporous scaffold is from 0.3 kPa to 1 kPa measured by the young modulus obtained from consecutive compression assays, as shown in the examples below.

Any drug or bio-active agent may be incorporated into the capsules or implants of the present invention provided that it does not interfere with the required functions of the encapsulated cells. Examples of suitable drugs or bio-active agents may include, without limitation, thrombo-resistant agents, antibiotic agents, anti-tumor agents, antiviral agents, anti-angiogenic agents, pro-angiogenic agents, anti-inflammatory agents, cell cycle regulating agents, their homologs, derivatives, fragments, pharmaceutical salts and combinations thereof. In some embodiment, the scaffolds may include angiogenic agents, such as VEGF, to promote vascular growth around the implants thereby facilitating the arrival of oxygen and nutrients to the implanted cells. In some embodiment, the scaffold may include anti-inflammatory drugs, such as steroidal anti-inflammatories.

Thus, in a particular embodiment, the capsule or the implant of the invention further comprises an anti-inflammatory agent, an antibiotic, a pro-angiogenic factor, or a combination thereof. In a particular embodiment, the pro-angiogenic factor is VEGF.

As mentioned above, in a third aspect it is provided the capsule or the implant of the invention for use in therapy, diagnosis or prognosis.

Encapsulated cells can be administered, e.g., injected or transplanted, into a patient in need thereof to treat a disease or disorder. In some embodiments, the disease or disorder is caused by or involves the malfunction of hormone- or protein-secreting cells in a patient. In these embodiments, hormone- or protein-secreting cells are encapsulated and administered to the patient. For example, encapsulated islet cells can be administered to a patient with diabetes. In other embodiments, the cells are used to repair tissue in a subject. In these embodiments, the cells form structural tissues, such as skin, bone, cartilage, muscle, or blood vessels. In these embodiments, the cells are preferably stem cells or progenitor cells.

A non-limiting list of diseases or disorders that can be treated with the capsules and implant of the invention include neurodegenerative diseases, such as Alzheimer's disease, Huntington's Disease, or Parkinson's Disease; cardiovascular diseases; metabolic diseases, such as diabetes type I and type II, liver failure, disorders of amino acid metabolism, disorders of organic acid metabolisms, disorders of fatty acid metabolism, disorders of purine and pyrimidine metabolism, lysosomal storage disorders, and disorders of peroxisomal metabolism; inflammatory disease; and cancer, including non-solid cancers and solid cancers,

Thus, in a particular embodiment of the third aspect, optionally in combination with any of the embodiments provided above or below, the capsule or the implant of the invention is for use in the treatment of a metabolic disease. In a more particular embodiment, the metabolic disease is diabetes. In an even more particular embodiment, the capsule or implant is for use in the treatment of diabetes type I.

In another embodiment, optionally in combination with any of the embodiments provided above or below, the capsule or the implant of the invention is for use in the treatment of a hepatic disease (i.e. a liver disease). The invention can be used to treat any disease that involves any kind of liver dysfunction, for instance, chronic liver disease, hepatitis, cirrhosis, liver cancer, non-alcoholic fatty liver disease, Reye syndrome, Type I glycogen storage disease, or Wilson disease.

The capsules and implants herein provided can be administered or implanted alone or in combination with any suitable drug or other therapy. Such drugs and therapies can also be separately administered (i.e., used in parallel) during the time the capsules or implants are present in a patient. Although the disclosed capsules or implants reduce fibrosis and immune reaction, use of anti-inflammatory and immune system suppressing drugs together with or in parallel with the capsules or implants is not excluded. In preferred embodiments, however, the disclosed capsules or implants are used without the use of anti-inflammatory and immune system suppressing drugs. In preferred embodiments, fibrosis remains reduced after the use, concentration, effect, or a combination thereof, of any anti-inflammatory or immune system suppressing drug that is used falls below an effective level.

The capsules of the implants of the invention can also be used for in vitro diagnosis or prognosis of disease, for instance, by encapsulating cells from a patient to culture them in vitro and then perform functional tests on them, such as insulin secretion tests. The cells can be differentiated cells, reprogrammed cells, or transdifferentiated cells. These assays may be performed on microfluidic arrays that allow assay multiplexing.

As mentioned before, the invention also provides the use of the hydrogel capsule or the implant of the invention for the in vitro culture of cells.

The composition and structure of the capsules of the invention provide the optimal conditions for cell culture, in particular for cell aggregates or organoids.

As above mentioned, the invention also provides in a fifth aspect an ex vivo method for differentiating an undifferentiated cell to an islet cell, or alternatively, for transdifferentiating a differentiated cell to an islet cell, comprising the steps of (a) producing a hydrogel capsule of the invention wherein the cell is the undifferentiated or differentiated cell; (b) contacting the hydrogel capsule of (a) with a factor selected from the group consisting of KGF (keratinocyte growth factor), SANT1 ((4-Benzyl-piperazin-1-yl)-(3,5-dimethyl-1-phenyl-1H-pyrazol-4-ylmethylene)-amine), retinoic acid, and mixtures thereof.

In a particular embodiment of the fifth aspect, optionally in combination with any of the embodiments provided above or below, the step (b) comprises contacting the hydrogel capsule with KGF, SANT1, and retinoic acid in sequential culture steps. The skill in the art would know that there are various techniques to generate islet cells (i.e. beta cells), all of which could be applied to the capsules of the invention (see, for example, Felicia W. Pagliuca et al., "Generation of functional human pancreatic β cells in vitro", Cell. 2014, vol. 159(2), pp. 428-439).

In a particular embodiment of the fifth aspect, optionally in combination with any of the embodiments provided above or below, the step (b) further comprises contacting the hydrogel capsule with extracellular matrix (ECM) from pancreas. Pancreatic ECM can be obtained by various methods, such as the one disclosed in the examples below.

The optimal conditions for 3D culturing of cells provided by the capsules of the invention facilitate the differentiating and transdifferentiating processes, in particular when the resulting cells are aggregate-forming cells.

As above mentioned, the invention also provides a method for producing a hydrogel capsule as defined in in the first aspect, the method comprising the steps of:
(a) forming the first core layer comprising the protein, i.e. collagen, and the cell; (b) allowing non-covalent reticulation of the protein to form a hydrogel; and (c) submerging the hydrogel in a solution comprising the crosslinking agent, i.e. tannic acid.

In a particular embodiment of the sixth aspect, optionally in combination with any of the embodiments provided above or below, the step (a) comprises: (i) providing an electrospraying device with a nozzle; (ii) pumping a composition comprising the protein and the cell into the tube of the nozzle; (iii) allowing the droplets to fall into a superhydrophobic surface. The hydrophobic surface was characterized measuring the contact angle. The contact angle is defined as the angle formed by the intersection of the liquidsolid interface and the liquid-vapor interface (geometrically acquired by applying a tangent line from the contact point along the liquid-vapor interface in the droplet profile). More specifically, a contact angle less than 90° indicates that wetting of the surface is favorable, and the fluid will spread over a large area on the surface, defined as a hydrophilic surface; while contact angles greater than 90° generally means that wetting of the surface is unfavorable so the fluid will minimize its contact with the surface and form a compact liquid droplet, defined as a hydrophobic surface. For superhydrophobic surfaces, water contact angles are usually greater than 150°, showing almost no contact between the liquid drop and the surface. Thus, in a more particular embodiment, the droplets fall into a surface with a water contact angle greater than or equal to 150 °.

In a particular embodiment of the sixth aspect, optionally in combination with any of the embodiments provided above or below, the step (b) is performed by heat treatment. More in particular, the heat treatment is performed from 30 to 40 °C, from 35 to 40 °C, or at 37 °C. Even more in particular, the heat treatment is performed during 1 to 15 min, 2 to 12 min, or 3 to 10 min. In a particular embodiment, the step (b) is performed at 37 °C during 3 min or at 37 °C during 15 min.

In a particular embodiment, optionally in combination with any of the embodiments provided above or below, the step (c) is performed in a solution comprising tannic acid at a concentration from 0.1 to 10 % w/w, from 0.5 to 5 % w/w, from 0.5 to 2 % w/w, from 0.5 to 3 % w/w, or 1 % w/w. In a more particular embodiment, the step (c) is performed from 0.5 to 3 min, from 0.5 to 2 min, or 1 min. In an even more particular embodiment, the step (c) is performed in a solution comprising 1% w/w tannic acid for 1 min. In an even more particular embodiment, the step (c) is performed in a solution comprising 3 % w/w tannic acid for 1 min.

Also described herein is the use of the hydrogel capsule as defined in the first aspect or the implant as defined in the second aspect in an *in vitro* companion diagnostic method.

In a particular embodiment, the companion diagnostic method comprises (a) producing a hydrogel capsule as defined in the first aspect wherein the cell is a cell from a subject; (b) contacting the hydrogel capsule produced in (a) with a drug; and (c) determining the effective drug dose to treat the subject. The skill in the art would understand that the capsules and implants of the invention can be used in companion diagnostic methods for a wide variety of diseases, such as diabetes. In this case, isletcells from the subject can be encapsulated and in vitro tested for glucose response. Thus, the capsules and implants of the invention constitute a great advance in the field of personalized medicine.
The companion diagnostic method described herein can also be used to deciding or recommending to initiate a medical regimen in a subject, or for determining the efficacy of a medical regimen in a patient.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention.

### Examples

### 1. Hydrogel preparation

### Collagen (reference embodiment)

Collagen solution was prepared in cold conditions following the manufactured instructions. Briefly, sterile acid-soluble type I collagen from rat tail (Corning cat. no. 354249) at 8.43 mg/mL was dissolved with 10x PBS (Sigma-Aldrich, cat. no. P4417/100 TAB) at the ratio 1:10 and neutralized with 1M NaOH (PanReac-AppliChem cat. no. 131687.1210) in order to achieve a pH of 7.5. The resulting hydrogel solution was dissolved with RPMI 1640 medium (Gibco^{™} cat. no. 11875085) to reach the final concentration of 4 mg/mL. Then the hydrogel solution was poured in a cylindrical PDMS (Silicone elastomer) (DOW Corning cat. no. SYLGARD 184) mold of 8 mm diameter and 3 mm height. Collagen hydrogel was polymerized after 10 min at 37 °C. After polymerization the crosslinked hydrogel could be easy detached from the mold by submerging it in a pre-warmed 1 x PBS solution.

### Collagen crosslinked with tannic acid

To achieve a more stable mesh structure, tannic acid (TA) was used (Sigma-Aldrich cat. no. 403040-50G) as a crosslinking agent for collagen. This approach consisted on submerging the crosslinked cylindrical hydrogel in a 1 wt% tannic acid solution for 1 min-period. The polymerization occurs at the interface between the two materials, keeping the submersion time short it is possible to obtain a more reticulated hydrogel on the outside surface than in the inside part. Finally, the tannic acid crosslinked hydrogel was washed 3 times with 1x PBS solution with constant stirring during 10 min-period each wash.

### Collagen crosslinked with methacrylate (reference embodiment)

Collagen methacrylate was prepared by the reaction of type I rat tail collagen with methacrylic anhydride in a manner adapted from a methodology reported by William T. Brinkman et al., "Photo-Cross-Linking of Type I Collagen Gels in the Presence of Smooth Muscle Cells: Mechanical Properties, Cell Viability, and Function", Biomacromolecules, 2003, vol. 4 (4), pp 890-895 . Briefly, type I rat tail collagen at 9.5 mg/mL (Corning cat. no. 354249) was dissolved in 0.02 N acetic acid (Pan Reac AppliChem cat. no. 1310081612) at 4 °C overnight in constant stirring to produce 4 mg/mL solution. Methacrylic anhydride (Sigma-Aldrich cat. no. 276685) was added at the ratio of 2:1000 and vigorously stirred at 4 °C. After 24-h reaction period, the mixture was dialyzed against 0.02 N acetic acid for 48h at 4°C with frequent changes in dialyzate. Following the dialysis, the collagen methacrylate solution was frozen overnight and lyophilized for 72h, and finally resuspended in 0.02 N acetic acid at the final concentration of 8.43 mg/mL.

Then the Lithium phenyl-2,4,6-trimethylbenzoylphosphinate (LAP) photoinitiator (TCI EUROPE N.V. cat. no. L0290) was diluted in a 10X PBS solution at 1 % w/v. The methacrylate collagen stock solution was diluted in 10x PBS containing the photoinitiator at the ratio 1:10 and neutralized with NaOH 1M in order to achieve a pH of 7.5. The resulting hydrogel solution was dissolved with RPMI medium to reach the final concentration of 4 mg/mL.

Then the hydrogel solution was poured in a cylindrical PDMS mold of 8 mm diameter and 3 mm height. Collagen hydrogel was polymerized after 10 min at 37 °C and irradiated with UV light at 14.82 mW/cm2 for 96 seconds using a UVP crosslinker (AnaltitikJena G116427). After polymerization the crosslinked hydrogel could be easy detached from the mold by submerging it in a pre-warmed 1xPBS solution.

### Pancreatic extracellular matrix hydrogel (reference embodiment)

Human cadaveric pancreas was first cut into small cubic pieces. The tissue pieces were washed 3 times with Milli-Q water in constant stirring.

Decellularization was performed using 1% w/v Triton X-100 (Sigma-Aldrich CAS: 9002-93-1 cat. no. X100-100ML) in 1xPBS. Penicillin / Streptomycin (Thermofisher cat. no. 15140122) was then added at a final concentration of 50 I.U./mL. The tissue was decellularized for 48 hours with a solution change every 12 hours.

After the detergent treatment, the tissue was rinsed thoroughly with water, placed into a new sterile beaker and stirred for an additional 5 days in Milli-Q water. The solution was changed every 12 hours. Then, ECM was frozen overnight, lyophilized during 72h and milled to produce a fine powder.

To generate a hydrogel form, the resulting pancreas ECM powder was enzymatically digested using an acidic pepsin solution. Fresh acidic pepsin (Sigma-Aldrich cat. no. P6887-1G) was dissolved in 0.1 M HCl at 1 mg/ mL final concentration. The ECM powder was placed into a 5 mL vial (30mg each vial), and the pepsin solution was added into at the final concentration of 30 mg ECM / mL pepsin solution and stirred for 48 hours. After digestion, the liquid ECM was neutralized to physiological pH using 1 M NaOH and 0.1 M HCl and salt condition was adjusted with 10x PBS. The resultant pre-gel solution was able to polymerize upon incubation at 37 °C for 12h.

### 2. Hydrogel characterization

### NMR-¹ H characterization of collagen metachrylated

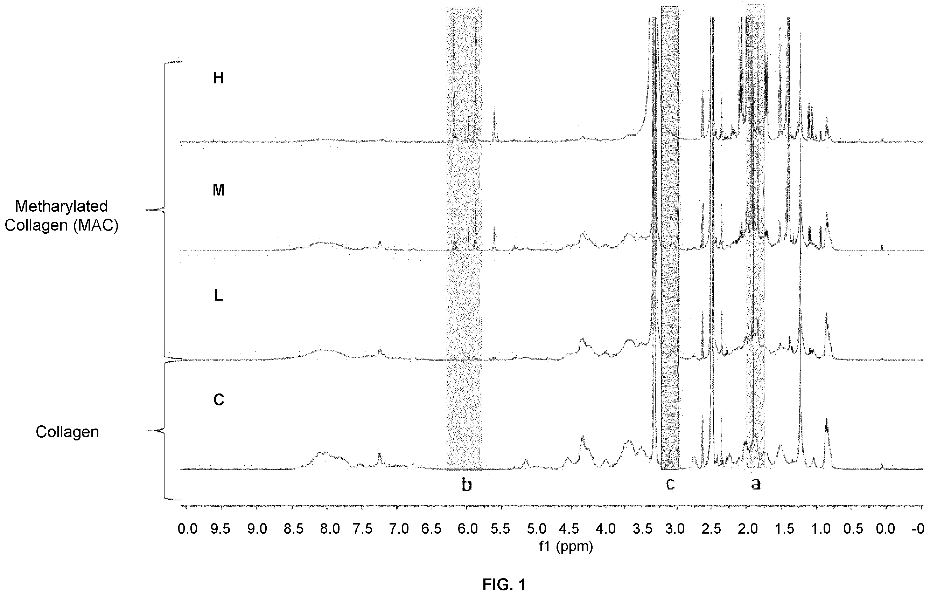

Characterization of functionalized collagen using ¹H-NMR spectroscopy displayed the presence of peaks between d = 5.3 and 5.5 ppm characteristic of the double bonds of acrylic protons of methacrylamides (see Figure 1). In addition, the signal at d = 1.8 ppm corresponds to the methyl group of the vinyl functional group. The signal at d = 2.89 ppm was assigned to the methylene hydrogen of lysine amines that was used as a reference signal to quantify the degree of modification. The degree of functionalization as gauged by NMR analysis was 79-81% of the available amine functionalities.

### Degradation

Cylinder-shaped hydrogels, 8 mm in diameter, were fabricated as described above for the degradation analysis. Hydrogels were placed in a 24 well-plateand and left swelling for 1 d submerged in 1 x PBS solution. A total of 3 mL of 0.25 U / mL of collagenase type I in 1 x PBS was added on the hydrogels and they were incubated at 37 °C, under 100 rpm shaking conditions. Then, hydrogels were weighed after 1, 2, 4, 24, 48h and 7 days. The percent hydrogel remaining (%Wr) was determined by the following equation: $% Wr = \frac{Wt}{Wi} ⋅ 100$

Here, Wt and Wi represents the weight of hydrogel composites after collagenase incubation and the initial weight after swelling.

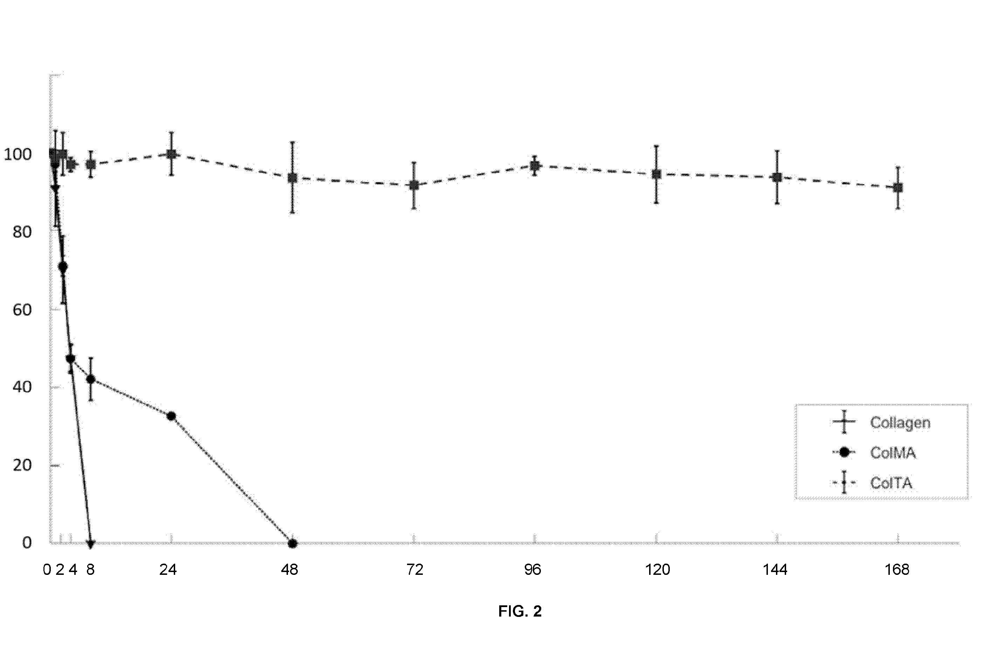

As shown in Figure 2, collagen crosslinking with methacrylate (CoIMA) had an improved resistance to collagenase degradation. Surprisingly, this resistance was greatly increased when collagen was instead treated with tannic acid (CoITA). Control hydrogel underwent degradation in 8 hours whereas ColMA underwent degradation in 48 hours showing a lower enzymatic degradation against collagenase. Collagen submerged in tannic acid (ColTA) did not show any degree of degradation over the 7 days of the experiment.

### SEM images

Cylinder-shaped hydrogels, 8 mm in diameter, were fabricated as described above for pore size quantification. Then, they were left swelling in Milli-Q water for 3 d. After that, dehydration was carried out by sequential immersion in graded ethanol solutions in Milli-Q water: 30%, 50%, 70%, 80%, 90%, and 96% v/v for 10 min each and twice for 100% ethanol. Then, samples were placed in the chamber of a critical point dryer (K850, Quorum technologies, UK), sealed, and cooled. Ethanol was replaced completely by liquid CO₂, and by slowly heating. This technique allowed dehydration of the hydrogels while avoiding their collapse. After critical point drying, hydrogels were covered with an ultra-thin coating gold and imaged by ultrahigh resolution scanning electron microscopy (SEM).

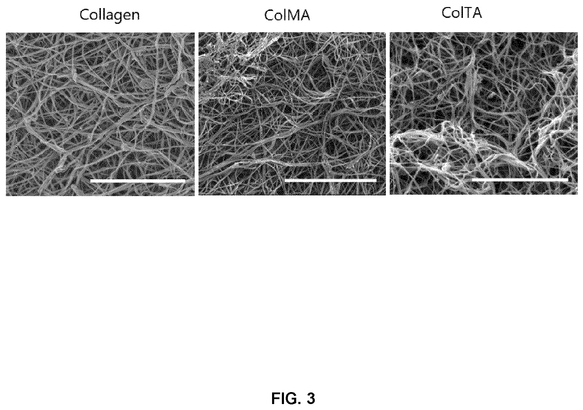

As shown in Figure 3, the biomaterial obtained is comprised of thin overlapping layers of fine interconnected fibrils. These interconnected fibrils formed porous in the nanoscale range. These nanopores allowed the diffusion of insulin hexamer across the hydrogel (5 nanometers diameter) and glucose molecule (1.5 nanometers diameter) and prevented the entrance of cells as macrophages which is in the range of micrometers (20-80 micrometers diameter).

### Stiffness

Mechanical properties of hydrogels were assessed using parallel plate rheometry (Discovery HR-2 rheometer, TA instruments, Inc., UK). Hydrogels were fabricated in cylindrical shape (1mm thick, 8 mm diameter) and bulk modulus (G') and viscous modulus (G") measurements were recorded at a frequency range of 1-10Hz at room temperature using 8mm aluminum plate geometry. The gap was adjusted starting from the original sample height and compressing the sample to reach a normal force of 0.3N. Rheological measurements were made on hydrogels after 24h post gelation.

The following table summarizes the storage modulus and the loss modulus of the different materials tested:

**Table 1**

| **Sample** | **Storage modulus (Pa)** | **Loss modulus (Pa)** |
|---|---|---|
| Col | 882 | 124 |
| ColMA | 187 | 37 |
| ColTA | 15184 | 1162 |

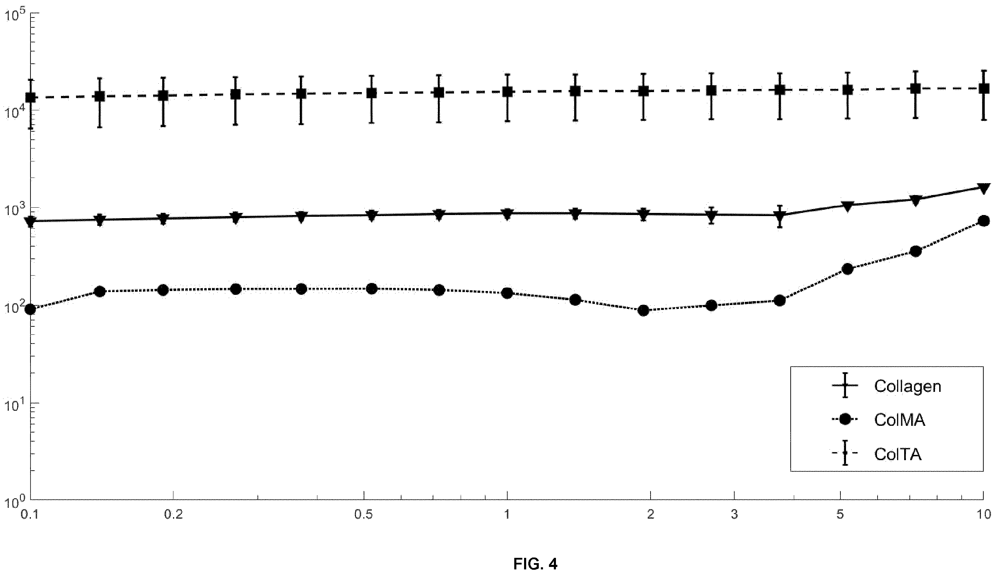

As shown in Figure 4, the storage modulus value increased from 182 Pa from ColMa hydrogels to 15184 Pa from ColTA. The storage modulus represents the elastic portion of the material or the stiffness. The energy dissipated as heat, represents the viscous portion, and is shown as the loss modulus. The frequency dependent measurements of the biomaterials from all formulations showed that the storage modulus was always higher than the loss modulus, showing that all hydrogels were predominantly elastic.

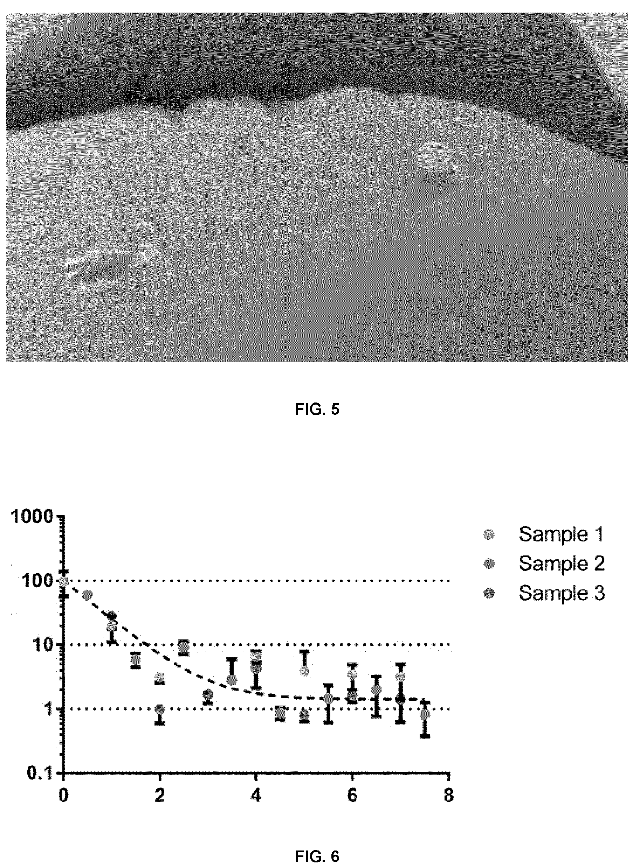

Figure 5 shows that the capsules of the invention present a perfectly round morphology and can be easily manipulated. This perfect morphology provides a great advantage for cell encapsulation because it minimizes the usual limitation of nutrients and hormones diffusion inside the capsules, therefore reducing cell necrosis.

### AFM (atomic force microscope) characterization

The collagen type I hydrogel solutions were prepared using rectangular-shaped PDMS mould of 1 cm x 0.5 cm. A volume of 160 ul of the hydrogel solution at 4 mg/mL were poured into each mould and allowed to polymerize for 30 min at 37° C. After polymerization, the rectangular PDMS moulds were lengthened in order to create a pool in one of the small sides of the rectangle. These pools were filled with tannic acid solution at 1% wt/v for 1 min, performing a gradient of crosslinking in the hydrogel. Then the hydrogel was demoulded and rinsed with PBS 1x three times.

The microrheology of the hydrogels was probed with an Atomic Force Microscope (AFM) mounted on the stage of an inverted optical microscope (Nikon Eclipse Ti-U). Pyrex-Nitride triangular cantilevers with force constant of 0.08 N/m, resonance frequency of 17 kHz and cantilever length of 200 µm (Nanoworld innovative technologies, PNP-TR-50) were used to analyze samples. AFM measurements were carried out at room temperature on cultured glass cover slips. The relationship between photodiode signal and cantilever deflection was calibrated before measurements. The calibration factor was taken as the slope of the linear relationship between the photodiode and position sensor signals recorded with the cantilever in contact with a bare region of the cultured glass cover slip. The force (F) on the cantilever was computed using JPK Nanowizard Control software.

The inventors then analyzed the stiffness along the hydrogel to determine the crosslinked effect of the tannic acid solution. As shown in Fig. 6 in all the 3 samples stiffness decreased along the x axis confirming that tannic acid diffuses while crosslinks the hydrogel.

### Porous hydrogel sizes

Cylinder-shaped hydrogels were left swelling in Milli-Q water for 3 d. After that, dehydration was carried out by sequential immersion in graded ethanol solutions in Milli-Q water: 30%, 50%, 70%, 80%, 90%, and 96% v/v for 10 min each and twice for 100% ethanol. Then, samples were placed in the chamber of a critical point dryer (K850, Quorum technologies, UK), sealed, and cooled. Ethanol was replaced completely by liquid CO2, and by slowly heating. After critical point drying, hydrogels were covered with an ultra-thin coating gold and imaged by ultrahigh resolution scanning electron microscopy (SEM).

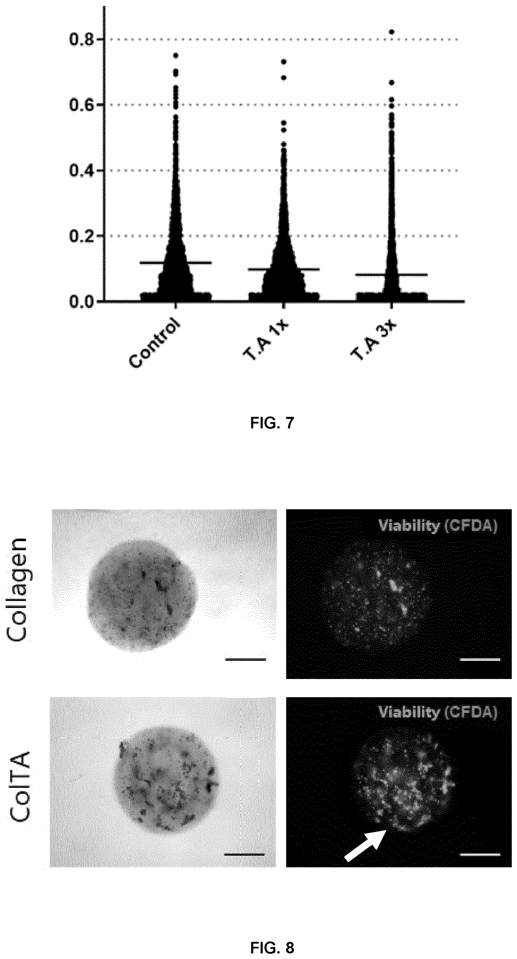

Quantification of porous diameter by ImageJ (Fig. 7) confirmed that all conditions presented diameter average size much smaller than T lymphocyte diameter. The average sizes of porous from Control, T.A 1x and T.A 3x, respectively were 0.118, 0.098 and 0.082 µm, whereas the T lymphocyte has sizes from 7 to 15 µm of diameter.

### Spheroid diameter sizes

Regarding the control of the spheroid sizes, the inventors studied how to generate spheres of up to 5 different diameters. By using different hydrogel volumes controlling the opening and closing time of the valve the inventors were able to successfully produce cell-laden spheroids in a range of sized from 1490 +/- 30 µm to 460 +/- 60 µm of diameter.

### 3. 3D spheroids bioprinting

3DDiscovery^{™} (regenHU Ltd., Switzerland) was used as a bioprinter platform, which is a versatile and cell friendly three-dimensional (3D) bioprinter that allows the fabrication of 3D structures in a working range of 130 x 90 x 60 mm. The bioprinter equipment includes a desktop instrument enclosed within a sterile hood and temperature control unit to ensure a constant temperature along the print head during the printing process.

The dispensing module is equipped with 3 different print heads namely time-pressure based, extrusion-based and inkjet/valve-based print heads. The print head used for the spheroid fabrication was the inkjet/valve printhead (Microvalve CF300, MVJ-D0.1S0.06) which incorporates a pneumatic valve that is automatically controlled to jet small amounts of low viscous materials in the nanolitre scale. Depending on the opening and closing valve time it is possible to control the volume of the deposited drop, and consequently the diameter of the final spheroid, as shown in the following table:

**Table 2**

| | | | | | |
|---|---|---|---|---|---|
| Opening time | 10.000 µs | Opening time | 50.000 µs | Opening time | 100.000 µs |
| Closing time | 10,000 µs | Closing time | 50.000 µs | Closing time | 100.000 µs |
| Diameter | 454 µm | Diameter | 539 µm | Diameter | 1095 µm |

Taking advantage of this technology, the inventors' strategy consists on the deposition of an array of drops over a super-hydrophobic surface. The pre-treated surface is able to maintain the spherical shape of the cell-laden hydrogel drop. With this methodology it is possible to fabricate automatically 100 spheroids/min.

### Superhydrophobic surface

Ultra-Ever Dry (SE 7.6.110) solvent based on two-part coating system (bottom and top) was used to prepare the superhydrophobic surfaces. To activate the surface, standard petri dishes (Thermofisher cat. no. 055061-INF) were washed 3 times with ultrapure water and 1 time with ethanol (PanReac AppliChem cat. no. 131085.1212). Each component was poured into two dedicated sprayers. First component was mixed and applied to the petri dishes during 5 second until a thin wet coating was formed. The Petri dishes were dry at room temperature during 15 to 20 minutes, then the second component was applied. The coating became superhydrophobic after 30 minutes of the second component coat application.

### Cell-laden bioink preparation

HFF 10.3 human fibroblast were directly reprogrammed (Sara Cervantes et al., "Late-stage differentiation of embryonic pancreatic β-cells requires Jarid2", Sci Rep. 2017, vol. 14;7(1), pp.11643), into beta-like cells purchased from IDIBAPS and expanded in a RPMI 1640 medium (Gibco^{™} cat. no.11875085) supplemented with 10% fetal bovine serum (FBS) (Thermofisher cat. no. 16000044) and 1% penicil/streptomycin (P/S) (Thermofisher cat. no. 15140122) at 37 °C and 5% CO₂ atmosphere. The β-like cells were dissociated to single cells using 0.05% Trypsin - 0.25% EDTA (Sigma-Aldrich cat. no. T4049-100ML) for 5 min at 37°C and placed in a 2mL sterile Eppendorf. The β-like cells were then centrifuged at 1200 r.p.m for 3 min to induce cell pellet formation into the bottom of the well.

To fabricate the cell-laden hydrogel, one volume of the cold collagen solution at 4 mg/mL concentration was mixed with the cell pellet to a final density of 1 x 10⁶ cells/mL. The bioprinter cartridge/syringe was filled with the cold collagen bioink and loaded into the bioprinter dispensing module. Square array pattern consisting in 50 points were designed using the BioCAD v1.0 software (regenHU Ltd., Switzerland), and launched to the bioprinter platform. The optimal printability was achieved at 6 °C using a 0.1 mm nozzle diameter, 0.2 bar of pressure and a valve opening time and closing time of 50000 µs. After printing, the petri dishes were placed at 37 °C for 3 minutes. Then, a more stable gelation was further achieved by cross-linking with tannic acid solution at 1 wt% submerging the spheroids for 1 minute. Then the spheroids were placed in a 24 non-treated MW plate (Costar cat. no. 3738) and washed 3 times in constant stirring with the RPMI medium. Afterwards, the spheroids were cultured in 3D suspension maintaining the 48 MW plate in constant light stirring.

### Cell-laden hepatocytes bioink preparation

The AML12 (alpha mouse liver 12) cell line was established from hepatocytes from a mouse (CD1 strain, line MT42) and purchased from ATCC (ATCC^{®}, cat. no. CRL-2254^{™}). These cells exhibit typical hepatocyte features such as peroxisomes and bile canalicular like structure. AML12 cells retain the capacity to express high levels of mRNA for serum (albumin, alpha 1 antitrypsin and transferrin) and gap junction (connexins 26 and 32) proteins and contain solely isoenzyme 5 of lactate dehydrogenase.

The base medium for this cell line is DMEM:F12 Medium supplemented of with 10% fetal bovine serum (FBS) (ATCC^{®} cat. No. 30-2020), 1% penicil/streptomycin (P/S) (Thermofisher, cat. No. 15140122) and Insulin-Transferrin-Selenium (ITS -G) (Thermofisher, cat. No. 41400045) called complete medium (CM).

These cells were cultured at 37 °C and 5% CO2 atmosphere. Prior each experiment, the AML12 cells were washed twice with Phosphate-Buffered Saline (PBS), detached from the flask (Corning^{®} cat. No. CLS430641) using 1 mL of 0.05% Trypsin - 0.25% EDTA (Sigma-Aldrich cat. No. T4049-100ML) for 5 min at 37°C and collected in 15 mL tubes falcon (Thermofisher, cat. No. 11507411) with 9 mL of CM in order to deactivate the trypsin. After that, the cells were centrifuged at 1200 r.p.m for 3 min to induce cell pellet formation into the bottom of the tube, washed with PBS and counted.

To fabricate the cell-laden hydrogel, one mL of the cold collagen solution at 4 mg/mL concentration as described previously in the section "AFM characterization", was mixed with the cell pellet to a final density of 1 x 10⁶ cells/mL. The bioprinter cartridge/syringe was filled with the cold collagen bioink and loaded into the bioprinter dispensing module. Square array pattern consisting in 50 spots were designed using the BioCAD v1.0 software (regenHU Ltd., Switzerland), and launched to the bioprinter platform. The optimal printability was achieved at 6 °C using a 0.1 mm nozzle diameter, 1 bar of pressure and a valve opening time and closing time of 50000 or 100000 µs. After printing, the petri dishes were placed at 37 °C for 3 minutes. Then, a more stable gelation was further achieved by cross-linking with tannic acid solution at 1% w/V submerging the spheroids for 1 minute. Then the spheroids were placed in a 24 non-treated MW plate (Costar cat. No. 3738) and washed 3 times in constant stirring in CM. Afterwards, the spheroids were cultured in 3D suspension maintaining the 48 MW plate in constant light stirring.

### Rat pancreatic β-cell bioprinting

The rat pancreatic β-cell line INS1E cells provided by August Pi i Sunyer Biomedical Research Institute (IDIBAPS), were cultured in RPMI R8758 medium (Sigma-Aldrich) (11.1mM glucose) supplemented with 10mM HEPES, 2mM L-glutamine, 1mM sodiumpyruvate, 0.05mM de 2-mercaptoethanol,10% fetal bovine serum (FBS) (v/v) (Thermofisher) and 1% penicillin/streptomycin (Thermofisher) at 37 °C and 5% CO₂ atmosphere. The β-cells were dissociated to single cells using 0.05% Trypsin - 0.25% EDTA (Sigma-Aldrich) for 3-4 min at 37°C, obtaining a cell pellet at the bottom of the well after a centrifugation. 3DDiscovery^{™} (regenHU Ltd., Switzerland) was used as a bioprinter platform. The print head used for the spheroid fabrication was the inkjet/valve printhead (Microvalve CF300, MVJ-D0.1S0.06. The bioprinter cartridge/syringe was filled with one volume of the cold collagen solution at 4 mg/mL concentration mixed with cell pellet, achieving a cell density of 7 x 10⁶ cells/mL. Square array pattern consisting in 50 points were designed using the BioCAD v1.0 software (regenHU Ltd., Switzerland), and launched to the bioprinter platform. The optimal printability was achieved at 6 °C using a 0.1 mm nozzle diameter, 0.2 bar of pressure and a valve opening time and closing time of 10 milliseconds which is equivalent to a diameter of 0.83 µm. After printing, hydrophobic petri dishes were placed at 37 °C for 15 minutes. Then the spheroids were immersed in a tannic acid solution of two different concentration 1x and 3x. Subsequently, the spheroids were placed in a 24 non-treated MW plate (Costar) and washed 3 times in constant stirring. Afterwards, the spheroids were cultured in 3D suspension in constant stirring, with low growth medium based on RPMI 1640 medium (Gibco^{™}) with low glucose (5.5mM) and 5% FBS.

### 4. Functional characterization of hydrogel capsules

### Cell viability

HFF 10.3 human cells were encapsulated in each hydrogel as described previously. The viability was studied after 1 and 7 d using the protocol for labeling cells with CFDA-SE assay kit (Astarte Biologics, Inc) and Hoechst. Cells suspension were prepared for labeling by determining volume necessary for 10⁷ cells per mL. A 10 mM solution of CFDA was prepared adding 90 ul of DMSO to one vial of CFDA and was mixed well. From this solution, 10 uL were removed and diluted in 10 mL of PBS or HBSS to make a 10 uM solution. Fainally the 10 uM solution was diluted 1:20 to make enough 0.5 uM CFDA to resuspend the cells at 10⁷ per mL. Cells were centrifuged to be labeled for 10 min at 200 x g, supernatant was decanted, and cell pellet was resuspended in 0.5 uM CFDA. Cells were incubated 15 min at 37°C to allow the dye to diffuse into the cells. Cells were centrifuged again, and pellet was resuspended in cell medium and incubated for 30 min at 37°C. After incubation, cells were centrifuged once more for 10 min at 200 x g. Supernatant was decanted and resuspend the cell pellet in culture medium to a concentration of 5 x 10⁶ per mL. Fluorescence images were captured using confocal microscopy and processed by FIJI software.

Figure 8 shows representative fluorescent images of living cells (green) within the cell-laden hydrogels of collagen and ColTA, after 7 days in culture. Both hydrogels showed high viability. Importantly, single beta-like cells gradually self-assembled and aggregated to form 3D spheroids within both hydrogels.

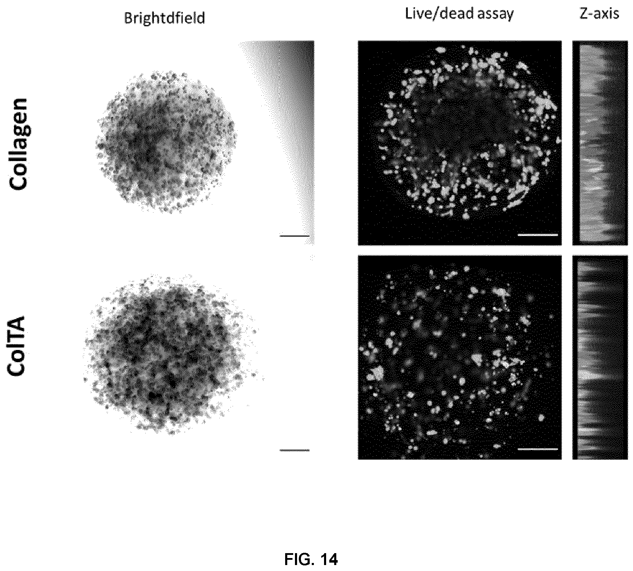

ML12 hepatocytes were encapsulated in 3D spheroid and the viability was assessed after 24 hours (Thermofisher, cat. No. L3224) according to the manufacturer's protocol. Briefly, the cells incapsulated within the hydrogels were cultured in CM for 24 hours. After that, the cells were washed with PBS three times for 5 minutes each followed by incubation with calcein AM (4 µM-green), ethidium homodimer-1 (2 µM-red) and Hoechst 33342 (Thermofisher, cat. No. 62249) in PBS for 25 minutes at room temperature and in the dark. Three washes with PBS for 5 minutes each were performed, and confocal images were taken (Figure 14). Both hydrogels showed high cell viability although the unrestricted collagen matrix promotes cell proliferation compared with collagen and tannic acid.

The viability of INS1E spheroids was measured using a LIVE/DEAD^{™} Viability/Cytotoxicity Kit (Thermo Fisher) according to the manufacturer's instructions. Dye solution was prepared by mixing 0,2% (v/v) ethidium homodimer-1, 0,05% (v/v) calcein AM and 0,1% (v/v) of Hoechest PBS1x. Fluorescence z-stack images were captured using confocal microscopy and processed by FIJI software.

This analysis demonstrated that cells within spheroids remained viable in all conditions, after cell encapsulation and bioprinting spheroid fabrication. This data demonstrate that tannic does not affect viability inside the spheroids.

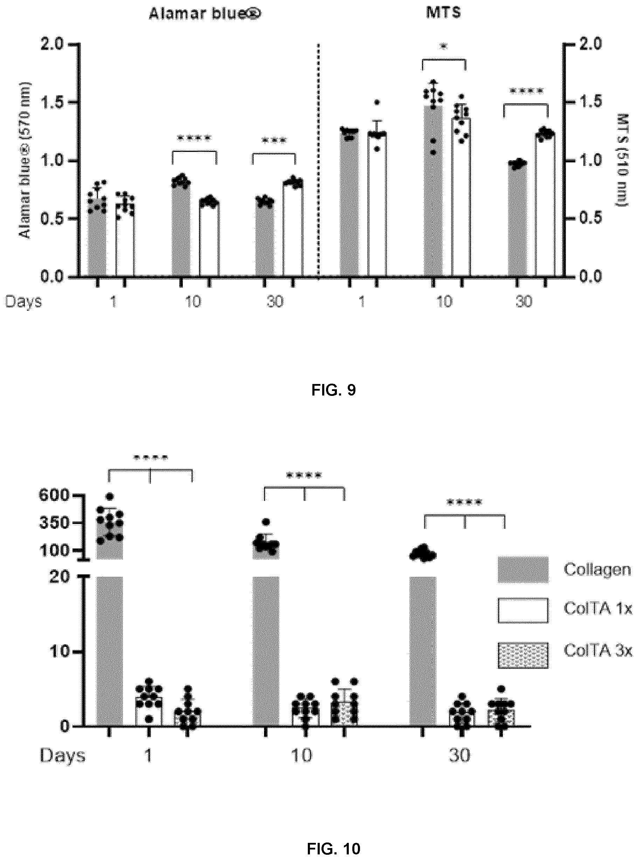

These results were corroborated by two different viability tests: Alamarblue^{®} and MTS (DAL1025 - Thermofisher and G3582 - Promega, respectively). These bioassays are based on redox indicators where the products are quantitatively related to cell proliferations. Each spheroid was placed in a 96-well plate throughout all the experiment (n=10). In case of MTS, 20 µL of MTS was added to 100 µL of medium and incubated for 3 hours and absorbance measured at 490 nm. For Alamarblue^{®} assay, 10 µL of reagents was mixed with 90 µL of medium and incubated for 3 hours in a black, flat bottom plate and fluorescence measured at 590 nm. Figure 9, shows the INS1E spheroids cultured up to 30 days. No difference in cell viability and metabolic activity was detected at day 1 between all the experimental conditions.

Surprisingly, the cells encapsulated in the hydrogel capsules treated with T.A. (tannic acid) showed enhanced proliferation compared to cells in control capsules (collagen with no T.A. treatment). These results clearly suggest that the capsules of the invention are suitable for long term cell encapsulation and that they provide an optimal environment for cells which improves their viability.

### Escaping assay

The same experimental set up described in the previous section for INS1E spheroids (single spheroids placed in 96 well-plate) was employed to evaluate the escaping cells from the spheroid. Specifically, the at day 1, 10 and 30, the spheroids were removed from the well and place in a new 96 well plate to avoid potentially proliferating cells escaped from spheroids and attached to the bottom of the well. The escaped cells were evaluated both in the supernatant and in the medium. For the supernatant, each sample was centrifuged at (1200 rpm for 10 min) but we did not count any cell in any of the experimental conditions under investigation. For the cells attached, 50 µL of trypsin-EDTA (0.025%) was employed was added to the well and incubated for 10 minutes was employed. The pellet was resuspended in in 10 µL and mix with 10 µL of trypan blue 0.4% (15250061, thermofisher) and counted using an automated cell counter Countess^{™} (15397802, fisher scientific). Figure 10 shows up to 600 cells/spheroids were able to escape from the collagen throughout all the experiment while few or no cells were found in both ColTA 1x and ColTA 3x conditions. Considerably, of few cells counted in ColTA 1x and ColTA 3x, more than 50% were dead.

### Insulin assay

Additionally, insulin secretion within the hydrogels was studied through the immunostaining. For this purpose, hydrogels were fixed in 10% formalin solution (Sigma-Aldrich) 14 d after fabrication. Then, hydro-gels were washed with PBS and cells were permeabilized with Block-Perm solution: 0.2% v/v Triton X-100 (Sigma-Aldrich) and 1% w/v BSA (Sigma-Aldrich) in PBS for 1 h. Afterward, hydrogels were washed in 1 x PBS and incubated with primary anti Insulin mouse Igg1 (Acris, cat. no. BM508) solution overnight at 4 °C. After washing 3 times with PBS, hydrogels were permeabilized with Block-Perm solution: 0.2% v/v Triton X-100 (Sigma-Aldrich) and 1% w/v BSA (Sigma-Aldrich) in PBS for 2 h. Then, incubated with secondary antibody, Alexa Fluor anti mouse IgG 647 (Invitrogen, cat. no. A32728) solution overnight at 4 °C. Hydrogels were washed 3 times with 1xPBS, mounted and stored at 4 °C before observation by confocal microscopy.

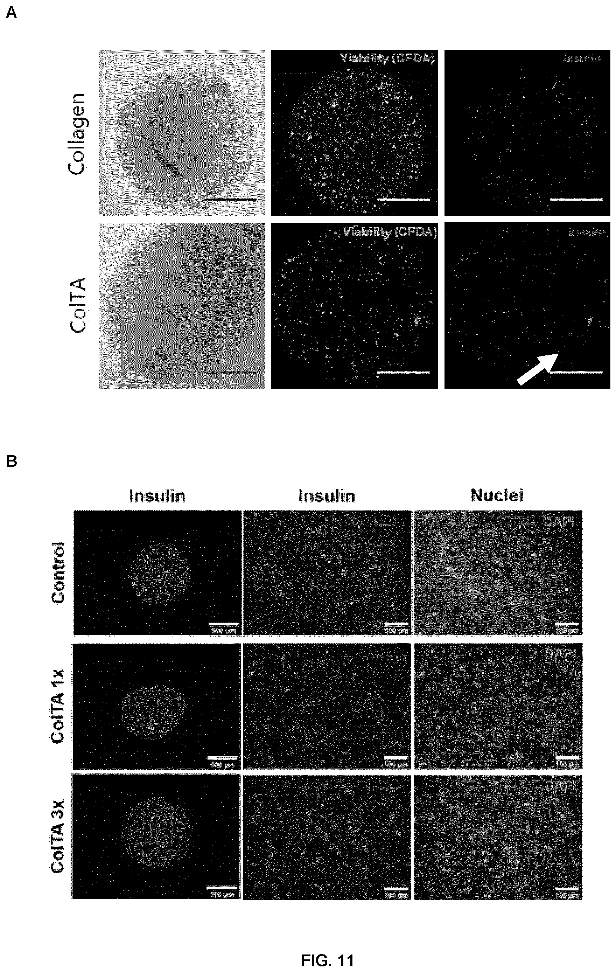

In Figure 11 A it is shown confocal images using immunofluorescent staining with antiinsulin antibody. Images showed that cells encapsulated in both biomaterials, collagen and ColTa, were functional and able to secrete insulin hormone (red color) after 7 days in culture. Figure 11 B shows further pictures using immunofluorescent staining with antiinsulin antibody in capsules treated with tannic acid at the indicated conditions.

Importantly, this data showed that most cells within spheroids produce insulin, and they were able to organize in functional clusters. The 4 different z-stacks from 3 spheroids per condition, show a homogeneous production of insulin in the whole spheroid. Thus, there were no differences in functionality depending on the position inside the spheroid. There were no qualitative differences between conditions, meaning that the treatment with Tannic Acid seems to not affect the functionality of cell embedded.

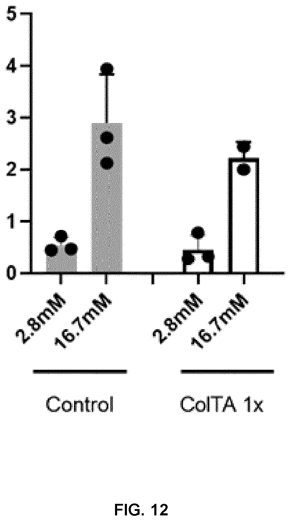

Glucose-stimulated insulin secretion assay was also measured by ELISA. Encapsulated β-cells at day 8 after fabrication, were preincubated with Krebs-Ringer bicarbonate HEPES buffer solution (115 mM NaCl, 24 mM NaHCO3, 5 mM KCL, 1 mM MgCa2·6H2O, 1 mM CaCl2·2H2O, 20 mM HEPES and 0.5% BSA, pH 7.4) containing 2.8mM glucose for 30 min. Then, spheroids were incubated at low glucose (2.8mM) for 1h followed by incubation at high glucose (16.7mM). After each incubation, supernatants were collected and cellular insulin contents were recovered in acid-ethanol solution. Insulin concentration was determined by ELISA following standard procedure (Figure 12). The results shows that the β-cells encapsulated in the capsules of the invention are capable of producing insulin.

### Albumin immunofluorescence staining

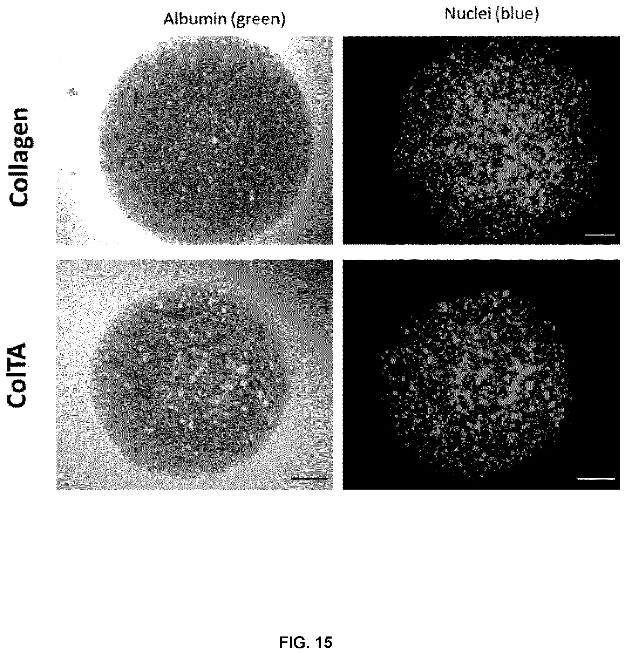

The cytosolic expression of albumin was detected using immunofluorescence technique as functional marker of healthy hepatocytes. For this purpose, hydrogels with hepatocytes prepared as described above, were kept in culture for 24 hours in CM and fixed using Formalin solution, neutral buffered, 10% (Sigma-Aldrich, cat. No. HT501128) for 1 hour. The cells were washed 3 times for 5 minutes under agitation. Sequentially, cells were permeabilized with Block-Perm solution: 0.2% v/v Triton^{™} X-100 (Sigma-Aldrich, cat. No. T8787) in PBS. The use of bovine serum albumin was avoided to not produce artefacts or undesired bindings. Afterward, hydrogels were washed with PBS 3 times for 5 minutes under agitation and incubated with primary anti-albumin antibody (Genetex, cat. No. GTX102419) overnight at 4 degrees in humidified chamber. The day after, the cells were incubated with secondary antibody, Alexa Fluor anti mouse IgG 647 (Invitrogen, cat. no. A32728) for 2 hours at room temperature. The Hydrogels were washed 3 times with PBS, counterstained with DAPI, mounted and stored at 4 degrees before observation by confocal microscopy (Figure 15). Interestingly, the further crosslinking due to tannic acid slow down the proliferation rate of the hepatocytes but at same time increase the differentiation state as demonstrated by high expression of albumin.

### Spheroid transplantation in vivo

NSG mices (n=9) were used to assess biodegradability of 3 different biomaterial conditions: Collagen crosslinked with tannic acid at 1% wt/vol and 3% wt/vol, and, for comparative purposes, collagen core covered with an alginate shell. For each condition 3 spheroids were transplanted per mice. The selected location was the bursa omentalis. The in vivo transplant was evaluated after 15 and 30 days.

Spheroids of 2 mm diameter were fabricated using collagen at 4 mg/mL. Tannic Acid (403040 Sigma Aldrich) solutions 1% and 3% wt/vol were prepared in PBS 1x. Then solutions were warmed-up and filtered with 0,22 ym filter (SLGP033RB Millex GP). Sodium alginate (W201502 Sigma Aldrich) powder was weighted and then sterilized in the UV for 15 minutes. Alginate was dissolved at 1.5% wt/vol in PBS 1x solution. Calcium chloride (C3306 Sigma Aldrich) powder was weighted and then sterilized in the UV for 15 minutes. Calcium chloride was dissolved at 2% wt/vol in Mili Q water.

On one hand, collagen spheroids were immersed in tannic acid solutions for 1 minute. Then 3 washes with PBS1x were done. In the other hand, to cover the collagen core with an alginate second layer, each spheroid was placed in a 96 well plate. Then, 20YI of alginate pre-polimeryzed with CaCl2 1:20 was added in each well. Finally, spheroids were immersed in a CaCl2 solution.

No toxicity was observed in all the implanted animals and the capsules were stable for all the experiment (i.e. for at least 30 days). Suprisingly, the capsules of the invention (i.e. crosslinked with tannic acid) produced significantly less fibrosis in the host tissue than the capsules with the alginate shell.

This experiment demonstrates that the capsules of the invention are suitable to be used in vivo for long periods of time with safety and without inflammatory or fibrotic effects in the host.

### 5. Preparation of microporous scaffold for embedding the 3D bioprinted hydrogel capsules

### Cryogel formation (Cellulose 0'5%)

To fabricate Carboxymethyl cellulose (CMC) cryogels at 0,5% (w/v), the inventors weighted 50 mg of CMC and the inventors diluted it into 5 ml of MilliQ water in a vial with stirring conditions, for further dilution down to 0,5% (w/v). Meanwhile the CMC was dissolving, the inventors prepared our molds. The molds consisted of a circular pool of PDMS with 1 mm high and 10 mm of diameter. On the bottom of it the inventors placed a squared 24x24 mm cover glass, and a rounded 12 mm diameter cover glass at the top, and we placed the molds into the fridge. Once the CMC was dissolved, the inventors prepared the crosslinking reagents; AAD will be at 50 mg/mL, MES buffer at 0,5M and pH at 5,5 and EDC at 1 ug/ml all dissolved in MilliQ water and vortexed to ensure the homogeneity in all the solution. To fabricate the prepolymer solution for 1 ml of CMC solution the inventors added 100 ul of MES buffer, 7 ul of AAD and 4 ul of EDC and vigorously pipetted to avoid early crosslinking before freezing. Then, the inventors filled the PDMS molds with the final prepolymer solution and the inventors put it fast into the freezer and we let it 24 hours. Next day, the inventors removed carefully the cover glasses and the PDMS mold and submerged into consecutive cleaning steps; 1x MilliQ water, 1x NaOH 100 Mm, 1x 10 mM EDTA, 1x MilliQ and 3x PBS. Once finished the cleaning protocol, the cryogels were sterilized for further cell seeding experiments in an autoclave.

Cryogels were placed in a 24-well plate (1 cryogel/well). 3D spheroids were seeded at a density of 100 or 1000 spheroids/cryogel in a small amount of DMEM/F12+0.5% BSA. After 15-20 min, 500 µL of the same medium was added and 3D spheroids were incubated at 37°C and 5% CO₂ for 2 or 3 days prior further experiments.

### 6. Microporous Scaffold characterization

### Pore analysis

For the pore analysis the fibers of the cryogel were stained adding 12 µl of 1mM fluoresceinamine in the previous prepolymer solution. Once stained, z-stack images were taken in a confocal microscope and the distribution of pore diameter can be quantified with ImageJ.

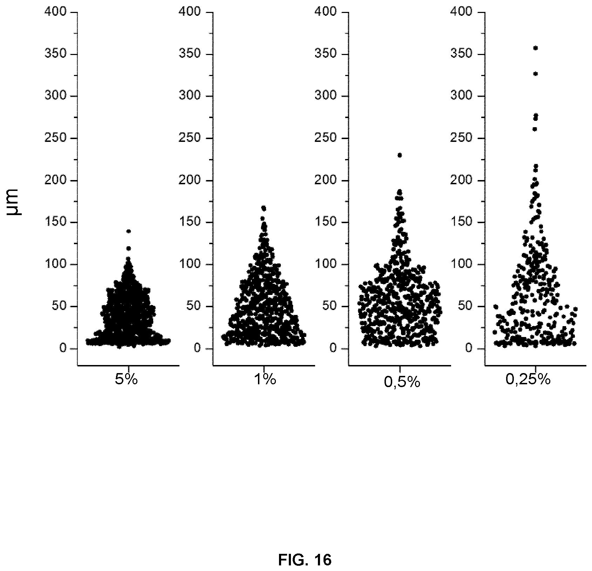

As can be seen in Figure 16 the pore distribution decreases as the concentration of material increases. Seeing the 5% graph, it is appreciated that the pore distribution reaches from 20 µm the smaller pores to 100 µm the bigger ones. In contrast, decreasing the amount of material to 0,25%, the smaller pores were around 20 µm, however the bigger pores can reach a diameter up to 250 µm. Observing the results found, the inventors chose to fabricate a smaller layer in the bottom with the higher concentration of material to generate a small porosity layer, and at the top generate a bigger distribution porosity that reaches up to 150 µm.

### Swelling

Swelling is the ratio of the amount of water uptake by a cryogel. To measure this, cryogels were fabricated as explained previously and after sterilizing, cryogels were dried at room temperature and weighted. Next, they were submerged into MilliQ water for 5 days, when they reached equilibrium where were weighted again. The swelling ratio was calculated as follows: $Swelling ratio = \frac{Weq - Wd}{Weq} ⋅ 100$

Where *W_{eq}* is the weight in equilibrium and *W_{d}* is the dry weight. In these experiments 3 measurements per cryogel and 3 cryogels per condition were weighted.

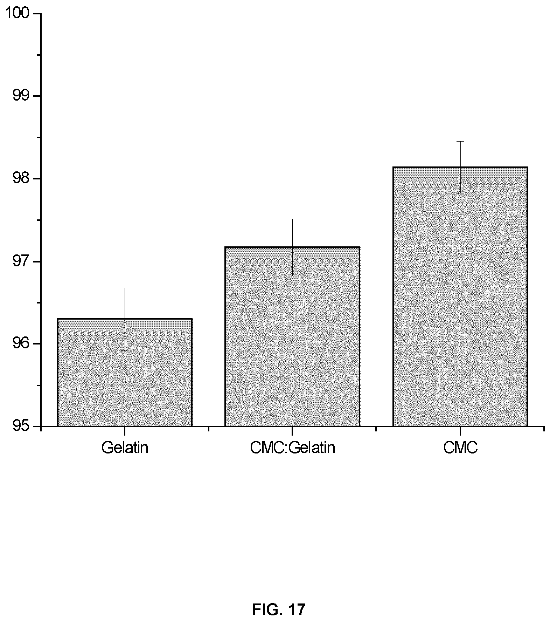

Figure 17 shows that with the same amount of material but changing the proportion of material the swelling ratio changes. In this case, the water uptake capacity of each cryogel composite depends on gelatin proportion. As it is observed in the Figure 17, the full CMC cryogel has swelling ratio of around 98%, however if gelatin is added, this swelling ratio decreases down to 96% in the full gelatin cryogel.

### Stiffness

Compression assays were performed to determine the stiffness of our samples. The compression assays were performed in a Zwick Z0.5 TN instrument (Zwick-Roell, Germany) with 5N load cell. The experiment was performed with samples at room temperature up to 30% final compression range at 0.1 mN of preloading force and at 20%/minute of strain rate. Finally, the young modulus was calculated from the slope of the range from 10% to 20% of compression. In these experiments 3 measurements per cryogel and 3 cryogels per condition were tested.

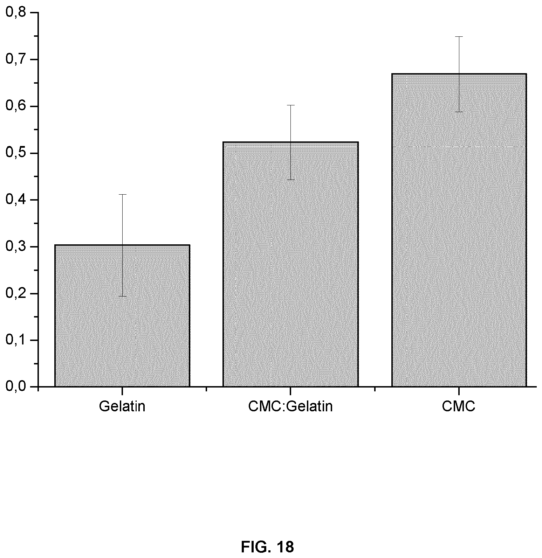

It is shown in Figure 18 that the stiffness results are related with the swelling results. As it is appreciated, the full CMC cryogels has a higher stiffness compared with full gelatine cryogel (0,7 kDa against 0,3 kDa). In that way, it can be said that the addition of gelatine decreases it's stiffness but without any variation of pore distribution.

### SEM Images

For SEM images, cryogels were fabricated as explained previously. After sterilizing, ethanol dehydration was done to substitute the water with ethanol. Consecutive washings were done by increasing the percentage of ethanol starting at 50%, and going up to 70%, 80%, 90%, 96% (x2) and 99,5% ethanol. Once all the water was substituted to ethanol, Critical point dry was done, in order to remove all the ethanol and replacing for CO₂. Then carbon sputtering was performed, and SEM images were taken.

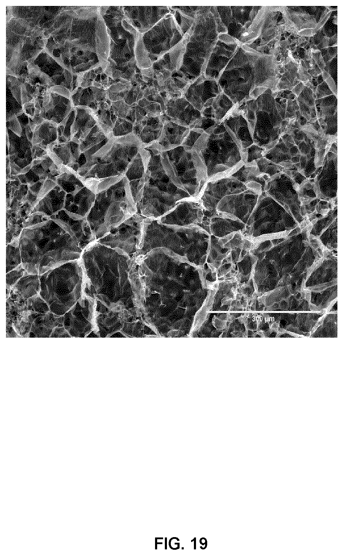

As it is shown in Figure 19, it is appreciated the high pore distribution in our. It can be seen that in the first layer there is this high pore heterogeneity, with areas with big pores and other areas with smaller pores.

### 7. Use of the implants for treating diabetic subjects

Insulin-dependent diabetic mellitus (T1DM) is an autoimmune disorder resulting from destruction of insulin-producing pancreatic β cells. The global burden associated with T1DM is from 5 to 10% of total diabetic patients, which account for 382 million of people. This amount is expected to rise to 592 million by 2035. Exogenous administration of insulin and tight blood glucose control are the recommended therapies to delay the progression of diabetes-associated complications and death. However, insulin does not provide efficient glucose control as functional pancreatic β cells islets do. In the last decade, major advances in β cell generation from pluripotent stem cells and somatic cells reprogramming have lifted great expectations for the development of patient-personalized pancreatic islets replacement therapies. However, pancreatic islet transplantation presents major limitations, such as immunosuppression, infection, and short-term therapy (limited viability of β cell). Here it is provided a new microencapsulation of 3D bioprinted pancreatic islets as artificial pancreas implantable in skeletal muscle tissue, which tackle efficiently these three limitations. In addition, the implantable microdevice will avoid continuous and invasive surgical interventions as it is required for the replacement of glucose sensors. For all this, the invention represents an important therapeutic option for T1DM treatment.

This invention is a novel approach to developing an islet transplantation scaffold as a β cell replacement therapy for T1DM, as it seeks to cover important gaps currently present in this therapeutic area. The main objective is to develop and customize a scaffold that will promote the engraftment of transplanted islet grafts and enable them to be retrieved at a later point, drawing on the latest knowledge in bioengineered materials. It can improve long-term graft revascularization, which until now has been a major stumbling block to clinical islet transplantation, by combining PEG and collagen, to create a structure ideal for supporting islets and newly formed vessels. By developing an innovative scaffold for islet transplantation, this invention seeks to innovate β cell replacement therapies for effectively treating T1D. Our work represents an important advancement toward making clinical islet transplantation an effective and safe means of treating T1D. This, in turn, holds potential for generating a positive social, clinical and economic impact by eliminating the burden of insulin therapy, reducing the direct and indirect costs associated with T1D and, most importantly, improving the quality of life of the millions affected by the disease worldwide.

Treatment of T1DM by multiple subcutaneous injections of exogenous insulin or subcutaneous pumps are unable to reproduce a physiological insulin profile. Moreover, it requires tight self-monitoring blood glucose level that in the long run does not protect against hypo and hyperglycemic events, impacting negatively on daily life and life expectancy of the patients. Although implantable artificial pancreas is going to be an alternative therapeutic option for T1DM treatment, it presents several limitations rooted in the fact of limited survival of implanted grafts as well as delay in glucose sensing and insulin production. On the other hand, short lifetime of glucose sensors and inadequate algorithms control make the pumps not a safe treatment option for T1DM patients. This invention represents an important step forward towards the creation of definitive treatment of T1DM, potentially saving millions of lives. The implantable artificial pancreas herein presented, tackle three important issues of most recent implantable pancreatic islets technology. First, the anatomical site of the implant here proposed, skeletal muscle, represents a safe and hypervascularized niche for optimal nutrient/oxygen supply as well as allows reversibility of the procedure extremely feasible. Second, the material here employed, in particular ColTA, and its pore size, 3 to 5 nm, cut notably the time between glucose sensing and the release of insulin. At same time, it provides efficient protection from host immune cells. Third, the pancreatic islets are embedded in biodegradable collagen which easily adapts itself to cellular clustering and growth. In addition, the cells are confined within a non- biodegradable tannic acid coating that prevents pancreas islets dispersion, but at same time does not affect the formation of new capillaries. This new complex system is the key to achieving better pancreatic islets performances, thanks to the integration of nanotechnology, biology and tissue engineering. Moreover, it is capable to self-regulating insulin release according to circulating glucose, providing high levels of stability and functionality overtime, yet resulting minimally invasive for the patients.

Thus, the hydrogel capsules or the implant of the invention comprising pancreatic islets are implanted in a tissue of a subject suffering from diabetes type I, for instance inside the skeletal muscle. The amount of islets per capsule and the amount of capsules to be implanted can be determined in view of various parameters, like disease severity, age, sex, etc. Importantly, the capsules of the invention can also be used to determine disease severity or drug response before treatment.

### Citation List

William T. Brinkman et al., "Photo-Cross-Linking of Type I Collagen Gels in the Presence of Smooth Muscle Cells: Mechanical Properties, Cell Viability, and Function", Biomacromolecules, 2003, vol. 4 (4), pp 890-895.
Felicia W. Pagliuca et al., "Generation of functional human pancreatic β cells in vitro", Cell. 2014, vol. 159(2), pp. 428-439
Zhou, Q., et al., "In vivo reprogramming of adult pancreatic exocrine cells to b -cells", 2008, Nature, vol. 455(7213), pp. 627-32.
Sara Cervantes et al., "Late-stage differentiation of embryonic pancreatic β-cells requires Jarid2", Sci Rep. 2017, vol. 14;7(1), pp.11643)

## Claims

1. A hydrogel capsule comprising:
- a cell;
- a protein; and
- a cross-linking agent;
wherein the cell is within a first core layer comprising the protein;
wherein the first core layer is surrounded by a second layer comprising the protein and the cross-linking agent; and
wherein the cross-linking agent is tannic acid and the protein comprises collagen.

2. The hydrogel capsule according to claim 1, wherein the cell is selected from the group consisting of pancreatic cell, hepatic cell, cardiovascular cell, nerve cell, muscle cell, cartilage cell, bone cell, skin cell, hematopoietic cell, immune cell, stem cell genetically engineered cell, reprogrammed cell, transdifferentiated cell, and mixtures therefor.

3. The hydrogel capsule according to claim 2, wherein the pancreatic cell is a beta cell.

4. The hydrogel capsule according to any of claims 1-3, wherein the cell is forming a cell aggregate or an organoid.

5. The hydrogel capsule according to any of claims 1-4, wherein the porous size of the second layer of the capsules is from 50 nm to 5 µm.

6. The hydrogel capsule according to any of claims 1-5, wherein the capsules have a mean diameter from 200 µm to 3 mm.

7. An implant comprising the hydrogel capsule according to any of claims 1-6 and a microporous scaffold.

8. The implant according to claim 7, wherein the microporous scaffold comprises a polymer selected from the group consisting of polysaccharide, collagen, gelatin, polyphosphazene, polyethylene glycol, poly(acrylic acid), poly(methacrylic acid), copolymer of acrylic acid and methacrylic acid, poly(alkylene oxide), poly(vinyl acetate), polyvinylpyrrolidone, and mixtures thereof.

9. The hydrogel capsule according to any of claims 1-6 or the implant according to any of claims 7-8 for use in therapy, diagnosis or prognosis.

10. The hydrogel capsule or the implant for use according to claim 9, which is for use in the treatment of diabetes type I.

11. Use of the hydrogel capsule as defined in any of claims 1-6 or the implant as defined in any of claims 7-8 for the in vitro culture of cells.

12. An ex vivo method for differentiating an undifferentiated cell to an islet cell, or alternatively, for transdifferentiating a differentiated cell to an islet cell, comprising the steps of:
(a) producing a hydrogel capsule as defined in any of claims 1-6 wherein the encapsulated cell is the undifferentiated or differentiated cell;
(b) contacting the hydrogel capsule produced in (a) with a factor selected from the group consisting of KGF, SANT1, retinoic acid, and mixtures thereof.

13. A method for producing a hydrogel capsule as defined in any of claims 1-6, the method comprising the steps of:
(a) forming the first core layer comprising the protein and the cell;
(b) allowing non-covalent reticulation of the protein to form a hydrogel; and
(c) submerging the hydrogel in a solution comprising the crosslinking agent.

## Patentansprüche

1. Eine Hydrogelkapsel umfassend:
- eine Zelle;
- ein Protein; und
- ein Vernetzungsmittel;
wobei sich die Zelle innerhalb einer ersten Kernschicht befindet, die das Protein umfasst; wobei die erste Kernschicht von einer zweiten Schicht umgeben ist, die das Protein und das Vernetzungsmittel umfasst; und
wobei das Vernetzungsmittel Gerbsäure ist und das Protein Kollagen umfasst.

2. Die Hydrogelkapsel nach Anspruch 1, wobei die Zelle ausgewählt ist aus der Gruppe bestehend aus Pankreaszelle, Leberzelle, kardiovaskulärer Zelle, Nervenzelle, Muskelzelle, Knorpelzelle, Knochenzelle, Hautzelle, hämatopoetischer Zelle, Immunzelle, Stammzelle, gentechnisch veränderter Zelle, umprogrammierter Zelle, transdifferenzierter Zelle und Mischungen davon.

3. Die Hydrogelkapsel nach Anspruch 2, wobei die Pankreaszelle eine Betazelle ist.

4. Die Hydrogelkapsel nach einem der Ansprüche 1 bis 3, wobei die Zelle ein Zellaggregat oder ein Organoid bildet.

5. Die Hydrogelkapsel nach einem der Ansprüche 1 bis 4, wobei die poröse Größe der zweiten Schicht der Kapseln von 50 nm bis 5 µm beträgt.

6. Die Hydrogelkapsel nach einem der Ansprüche 1 bis 5, wobei die Kapseln einen mittleren Durchmesser von 200 µm bis 3 mm haben.

7. Ein Implantat, das die Hydrogelkapsel nach einem der Ansprüche 1 bis 6 und ein mikroporöses Gerüst umfasst.

8. Das Implantat nach Anspruch 7, wobei das mikroporöse Gerüst ein Polymer umfasst, das aus der Gruppe ausgewählt ist, die aus Polysaccharid, Kollagen, Gelatine, Polyphosphazen, Polyethylenglykol, Poly(acrylsäure), Poly(methacrylsäure), Copolymer von Acrylsäure und Methacrylsäure, Poly(alkylenoxid), Poly(vinylacetat), Polyvinylpyrrolidon und Mischungen davon besteht.

9. Die Hydrogelkapsel nach einem der Ansprüche 1 bis 6 oder das Implantat nach einem der Ansprüche 7 bis 8 zur Verwendung in der Therapie, Diagnose oder Prognose.

10. Die Hydrogelkapsel oder das Implantat zur Verwendung nach Anspruch 9, welches zur Verwendung bei der Behandlung von Diabetes Typ I bestimmt ist.

11. Verwendung der Hydrogelkapsel wie in einem der Ansprüche 1 bis 6 definiert oder des Implantats wie in einem der Ansprüche 7 bis 8 definiert für die In-vitro-Kultur von Zellen.

12. Ein Ex-vivo-Verfahren zum Differenzieren einer undifferenzierten Zelle zu einer Inselzelle oder alternativ zum Transdifferenzieren einer differenzierten Zelle zu einer Inselzelle, umfassend die folgenden Schritte:
(a) Herstellen einer Hydrogelkapsel wie in einem der Ansprüche 1 bis 6 definiert, wobei die eingekapselte Zelle die undifferenzierte oder differenzierte Zelle ist;
(b) Inkontaktbringen der in (a) hergestellten Hydrogelkapsel mit einem Faktor, der aus der Gruppe ausgewählt ist, die aus KGF, SANT1, Retinsäure und Mischungen davon besteht.

13. Ein Verfahren zur Herstellung einer Hydrogelkapsel wie in einem der Ansprüche 1 bis 6 definiert, wobei das Verfahren die folgenden Schritte umfasst:
(a) Bilden der ersten Kernschicht, die das Protein und die Zelle umfasst;
(b) Ermöglichen einer nicht-kovalenten Vernetzung des Proteins, um ein Hydrogel zu bilden; und
(c) Eintauchen des Hydrogels in eine Lösung, die das Vernetzungsmittel umfasst.

## Revendications

1. Une capsule d'hydrogel comprenant :
- une cellule ;
- une protéine ; et
- un agent de réticulation ;
dans laquelle la cellule se trouve à l'intérieur d'une première couche centrale comprenant la protéine ;
dans lequel la première couche centrale est entourée d'une seconde couche comprenant la protéine et l'agent de réticulation ; et
dans lequel l'agent de réticulation est l'acide tannique et la protéine comprend le collagène.

2. La capsule d'hydrogel selon la revendication 1, dans laquelle la cellule est choisie dans le groupe constitué par une cellule pancréatique, une cellule hépatique, une cellule cardiovasculaire, une cellule nerveuse, une cellule musculaire, une cellule cartilagineuse, une cellule osseuse, une cellule de la peau, une cellule hématopoïétique, une cellule immunitaire, une cellule souche, une cellule génétiquement modifiée, une cellule reprogrammée, une cellule transdifférenciée et des mélanges de celles-ci.

3. La capsule d'hydrogel selon la revendication 2, dans laquelle la cellule pancréatique est une cellule bêta.

4. La capsule d'hydrogel selon l'une quelconque des revendications 1 à 3, dans laquelle la cellule forme un agrégat cellulaire ou un organoïde.

5. La capsule d'hydrogel selon l'une quelconque des revendications 1 à 4, dans laquelle la taille poreuse de la seconde couche des capsules est de 50 nm à 5 µm.

6. La capsule d'hydrogel selon l'une quelconque des revendications 1 à 5, dans laquelle les capsules ont un diamètre moyen de 200 µm à 3 mm.

7. Un implant comprenant la capsule d'hydrogel selon l'une quelconque des revendications 1 à 6 et un échafaudage microporeux.

8. L'implant selon la revendication 7, dans lequel l'échafaudage microporeux comprend un polymère choisi dans le groupe constitué par un polysaccharide, le collagène, la gélatine, le polyphosphazène, le polyéthylène glycol, le poly(acide acrylique), le poly(acide méthacrylique), un copolymère d'acide acrylique et d'acide méthacrylique, le poly(oxyde d'alkylène), le poly(acétate de vinyle), la polyvinylpyrrolidone et des mélanges de ceux-ci.

9. La capsule d'hydrogel selon l'une quelconque des revendications 1 à 6 ou l'implant selon l'une quelconque des revendications 7 à 8 pour une utilisation en thérapie, diagnostic ou pronostic.

10. La capsule d'hydrogel ou l'implant pour l'utilisation selon la revendication 9, qui est destiné(e) à être utilisé(e) dans le traitement du diabète de type I.

11. Utilisation de la capsule d'hydrogel telle que définie dans l'une quelconque des revendications 1 à 6 ou de l'implant tel que défini dans l'une quelconque des revendications 7 à 8 pour la culture in vitro de cellules.

12. Un procédé ex vivo pour différencier une cellule indifférenciée en une cellule insulaire, ou, alternativement, pour transdifférencier une cellule différenciée en une cellule insulaire, comprenant les étapes consistant à :
(a) produire une capsule d'hydrogel telle que définie dans l'une quelconque des revendications 1 à 6, dans laquelle la cellule encapsulée est la cellule indifférenciée ou différenciée ;
(b) mettre en contact la capsule d'hydrogel produite en (a) avec un facteur choisi dans le groupe constitué par KGF, SANT1, l'acide rétinoïque et des mélanges de ceux-ci.

13. Un procédé de production d'une capsule d'hydrogel telle que définie dans l'une quelconque des revendications 1 à 6, le procédé comprenant les étapes consistant à :
(a) former la première couche centrale comprenant la protéine et la cellule ;
(b) permettre une réticulation non covalente de la protéine pour former un hydrogel ; et
(c) immerger l'hydrogel dans une solution comprenant l'agent de réticulation.
